(19) Europäisches Patentamt
European Patent Office
Office européen des brevets

(11) Publication number: **0 294 818 A2**

# (12) EUROPEAN PATENT APPLICATION

(21) Application number: 88109239.9

(22) Date of filing: 10.06.88

(51) Int. Cl.[4]: C07D 401/04 , C08K 5/34 , C07D 401/14

Claims for the following Contracting State: ES.

(30) Priority: 12.06.87 US 60878

(43) Date of publication of application: 14.12.88 Bulletin 88/50

(84) Designated Contracting States: BE CH DE ES FR GB IT LI NL SE

(71) Applicant: PENNWALT CORPORATION
Pennwalt Building Three Parkway
Philadelphia Pennsylvania 19102(US)

(72) Inventor: Myers, Terry N.
780 Maple Road
Williamsville New York 14221(US)

(74) Representative: Abitz, Walter, Dr.-Ing. et al
Abitz, Morf, Gritschneder, Freiherr von Wittgenstein Postfach 86 01 09
D-8000 München 86(DE)

## (54) Hindered amine light stabilizers containing aromatic carboxy groups and derivatives.

(57) There are provided hindered amine light stabilizers of the general formula

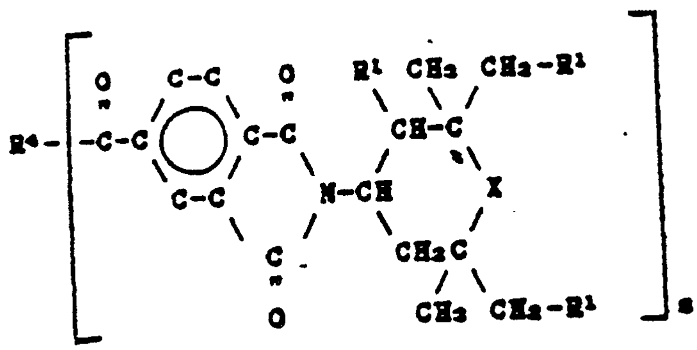

,

wherein the hindered amine is attached to the nitrogen of an imide which also is attached to an aromatic group which contains a carboxy group and wherein

$R^1$ is, e.g., hydrogen or substituted or unsubstituted aliphatic of 1-20 carbons;

X is

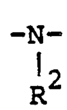 , 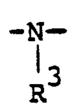 or 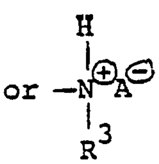 ;

$R^2$ is, e.g., oxyl, hydroxy or substituted or unsubstituted aliphatic acyl of 1-20 carbons;

$R^3$ is e.g., hydrogen or substituted or unsubstituted aliphatic of 1-20 carbons;

A is, e.g., halogen or sulfate; and

EP 0 294 818 A2

$R^4$ is, e.g., hydroxy, halogen or a substituted or unsubstituted, mono or polyfunctional alcohol, amine, mercaptan or molecular mixture thereof, the carboxy group being a site for preparing unique derivatives, both polymeric and non-polymeric, useful as additives for preventing degradation of polymers and copolymers.

# HINDERED AMINE LIGHT STABILIZERS CONTAINING AROMATIC CARBOXY GROUPS AND DERIVATIVES

## Background of the Invention

This invention relates to hindered amine light stabilizers in which the hindered amine is attached to the nitrogen of an imide group which is attached to an aromatic group containing a carboxylic acid or derivative thereof.

Hindered amine light stabilizers (hereinafter referred to as HALS) are a well-known class of compounds known to prevent the retard the degradation of polymers in which they are incorporated (Kirk-Othmer Encyclopedia of Chemical Technology, 3rd Ed., Vol 23, "UV Stabilizers", pp 615-627). Many patents on HALS additives and monomers have been issued, e.g., U.S. Patent 4,336,183. The fundamental HALS functionality is a rather small molecular ensemble, generally water soluble; this prohibits use of simple, low molecular weight HALS because they are readily leached from the polymer substrate upon exposure to moisture.

HALS imides such as N-(2,2,6,6-tetramethyl-4-piperidinyl)maleimide are known and have been used to prepare HALS-containing copolymers (as in U.S. Patent 4,569,997 and British Patent 2,145,100). Other N-(2,2,6,6-tetramethyl-4-piperidinyl)imides are disclosed in U.S. Patent 4,356,307, but the disclosed compounds are non-aromatic imides. N-(2,2,6,6-tetramethyl-4-piperidinyl)phthalimide and N,N′-bis-(2,2,6,6-tetramethyl-4-piperidinyl)pyromellitic diimide have been disclosed as polymer stabilizers in U.S. Patent 3,904,581, although neither compound was prepared nor tested.

No reference to the instant invention was found in the literature.

## Summary of the Invention

This invention is directed to compounds of general formula

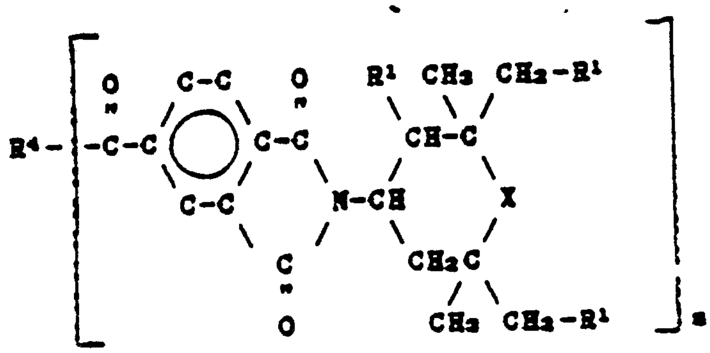

I

and derivatives thereof where $R^1$ is selected from hydrogen, substituted or unsubstituted aliphatic radical of 1 to 12 carbons and substituted or unsubstituted aromatic or araliphatic radical of 6 to 18 carbons;
X is selected from

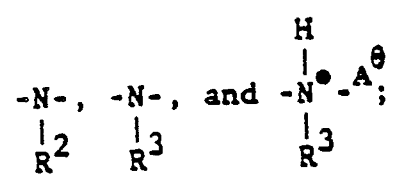

$R^2$ is selected from oxyl, hydroxy, and substituted or unsubstituted aliphatic acyl of 1-20 carbons, substituted or unsubstituted alicyclic acyl of 6-14 carbons, substituted or unsubstituted aryl acyl of 7-22

carbons, substituted or unsubstituted araliphatic acyl of 7 to 22 carbons, -C(=O)-N($R^1$)($R^5$) and -COOR$^5$; $R^3$ is selected from hydrogen, substituted or unsubstituted aliphatic of 1-20 carbons, substituted or unsubstituted araliphatic of 7 to 22 carbons, alkoxyalkyl of 2-21 carbons, and -$CH_2$-COOR$^5$; $R^4$ is selected from hydroxy, -$O^{\ominus}$, halogen or the residue from a substituted or unsubstituted, mono or polyfunctional alcohol, amine, mercaptan or molecular mixture thereof including hydroxy- and amine-containing polymers which may be in the backbone, pendant; and terminally functionalized; $R^5$ is selected from hydrogen, substituted or unsubstituted aliphatic of 1 to 20 carbons, substituted or unsubstituted aryl of 6-10 carbons, substituted or unsubstituted araliphatic of 7-22 carbons, and substituted or unsubstituted alicyclic of 5-12 carbons which may optionally contain heteroatom ring members selected from nitrogen, oxygen and sulfur; and s is an integer of 1-100.

A is selected from chloride, bromide, sulfate, acid sulfate, sulfite, acid sulfite, p-toluenesulfonate, phenylsulfonate, methylsulfonate, phosphate, acid phosphate, or carboxylate from any carboxylic acid, or $R^4$ when $R^4$ is -$O^{\ominus}$.

Substituents for the aliphatic, aryl, aliphatic and alicyclic radicals include one or more groups selected from alkyl of 1-4 carbons, alkoxy of 1-4 carbons, acyl or acyloxy of 1-12 carbons, alkoxycarbonyl of 2-5 carbons, arylcarbonyl of 7-11 carbons, acryloyloxy, methacryloyloxy, aryloxy of 6-10 carbons, aralkyl of 7-10 carbons, aryloxycarbonyl of 7-11 carbons, aryl of 6-10 carbons, amino, hydroxy, carboxy, nitrile, chloro, bromo, epoxy, alkyl mercapto of 1-4 carbons, aryl mercapto of 6-10 carbons, alkylamino of 1-4 carbons, dialkylamino of 2-8 carbons total, arylamino of 6-10 carbons, aryl alkyl amino of 7-10 carbons, and trialkoxysilyl of 3-9 carbons.

## DETAILED DESCRIPTION OF THE INVENTION

The compounds of this invention are characterized by the presence of at least one hindered amine group attached to the nitrogen of an imide which is also attached to an aromatic group, which also contains a carboxy group or a derivative thereof:

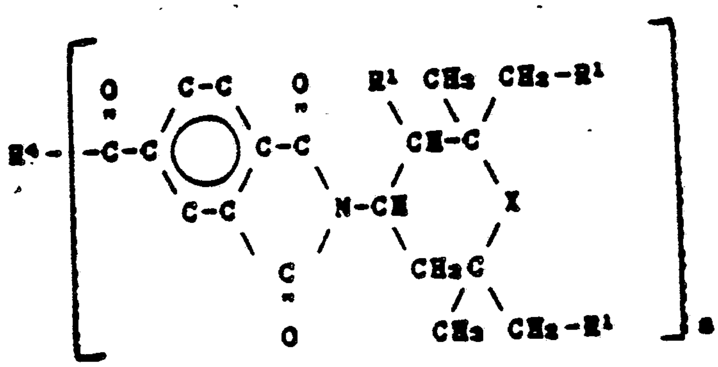

I

wherein $R^1$, $R^4$, s and X are as previously defined.

As a substituted or unsubstituted aliphatic radical of 1-20 carbons, $R^1$ is, for example, methyl, ethyl, isopropyl, n-hexyl, isododecyl, preferably hydrogen or alkyl of 1-4 carbons; most preferably hydrogen. As a substituted or unsubstituted aryl radical or araliphatic radical of 7-22 carbons, $R^1$ is, for example, phenyl, benzyl, tolyl, 4-butoxyphenyl, 2-methoxyphenyl, trimethylphenyl, 4-n-octylphenyl; preferably phenyl or tolyl, most preferably phenyl, hydrogen or methyl.

As a substituted or unsubstituted aliphatic acyl radical of 1-20 carbons, alicyclic acyl radical of 6-14 carbons, aryl acyl radical of 7-14 carbons, or aliphatic acyl radical with 7-22 carbons, $R^2$ is, for example, formyl, acetyl, chloroacetyl, acryloyl, 4-ethoxybenzoyl, benzoyl, toluoyl, cyclohexyl-1-carbonyl, 3-phenylpropionyl, crotonoyl, propionyl, butyryl, octanoyl, dodecanoyl, stearoyl, chlorobenzoyl, isopropylbenzoyl, 2,4-dichlorobenzoyl, 4-methoxybenzoyl, 3-butoxybenzoyl, 2-hydroxybenzoyl, 3,5-di-t-butyl-4-hydroxybenzoyl, beta-(3,5-di-t-butyl-4-hydroxyphenyl)propionyl, phenylacetyl, cinnamoyl, 1- or 2-naphthoyl or decahydronaphthoyl; preferably alkanoyl of 2-5 carbons, benzoyl, or 3,5-di-t-butyl-4-hydroxybenzoyl. As -C(=O)-N($R^1$)($R^5$), $R^2$ is, for example, methylcarbamoyl, n-butylcarbamoyl, dodecylcarbamoyl, dimethyl carbamoyl, diethylcarbamoyl or di-n-hexylcarbamoyl, piperidin-1-ylcarbonyl, piperazine-1-ylcarbonyl or morpholin-1-ylcarbonyl, phenylaminocarbonyl, (4-butylphenyl)aminocarbonyl, alpha-naphthyl-aminocarbonyl, N-phenyl-N-hexylaminocarbonyl, N-(trimethylphenyl)-N-amylaminocarbonyl, diphenylaminocarbonyl, di-(4-methylphenyl)aminocarbonyl, N-phenyl-N-(alpha-naphthyl)aminocarbonyl, or N-(4-benzylphenyl)-N-(phenyl)-aminocarbonyl.

As a substituted or unsubstituted aliphatic radical of 1-20 carbons or araliphatic radical of 7-22 carbons, $R^3$ is for example methyl, ethyl, n-propyl, n-pentyl, 2-bromoethyl, n-hexyl, n-octyl, n-decyl, n-dodecyl, allyl, methallyl, 2-butenyl, 2-hexenyl, 2-hydroxyethyl, 3-phenoxy-2-hydroxypropyl, 2,3-epoxypropyl, benzyl or propargyl; preferably alkyl of 1 to 4 carbons, or allyl, most preferably alkyl of 1 to 4 carbons. As an alkoxyalkyl of 2-21 carbons, the alkyl part of $R^3$ can contain 1-3 carbons and the alkoxy part can consist of 1-18 carbons, as in, for example, methoxymethyl, ethoxymethyl, 2-methoxyethyl, 2-ethoxyethyl, 2-n-butoxyethyl, 3-n-butoxypropyl, 2-octoxyethyl or 2-octdecyloxyethyl; preferably an alkoxyalkyl group with 2-6 carbons. As $-CH_2-C(=O)-OR^5$, $R^3$ is, for example, methoxycarbonylmethyl, butoxycarbonylmethyl, or (benzyloxy)carbonylmethyl.

As a substituted or unsubstituted aliphatic radical of 1-20 carbons, $R^5$ is, for example, methyl, ethyl, isopropyl, n-butyl, isobutyl, isopentyl, n-hexyl, n-octyl, 2-chloroethyl, 2-(dimethylamino)ethyl, n-decyl, n-dodecyl, allyl, 3-(trimethoxysilyl)propyl, 2-butenyl or 2-hexenyl; preferably alkyl of 1-4 carbons. As a substituted or unsubstituted alicyclic radical of 5-10 carbons with optional heteroatoms, $R^5$ is for example tetramethylpiperidinyl, cyclohexyl, trimethylcyclohexyl, cyclohexenyl, cyclooctyl; preferably cyclohexyl. As a substituted or unsubstituted aryl radical of 6-10 carbons or araliphatic radical of 7-22 carbons, $R^5$ is, for example, phenyl, naphthyl, benzyl, 2-phenoxyethyl, 4-vinylphenyl, alpha-phenylethyl, or cumyl; preferably phenyl or benzyl.

$R^4$ defines a derivative of the aromatic carboxylic acid group and as such must contain at least one oxygen, nitrogen, sulfur or halogen atom capable of bonding to the aromatic acyl group. When attached to an oxygen, nitrogen or sulfur of $R^4$, the invention comprises a compound containing one or more groups having the structure shown above incorporated as one or more ester, amide, thioester group or a mixture thereof as derived from a substituted or unsubstituted, mono or polyfunctional alcohol, amine, mercaptan or molecular mixture thereof, including hydroxy-and amine-containing polymers which may be backbone, pendant and terminally functionalized.

As a halogen $R^4$ can be, for example, chlorine or bromine; preferably chlorine.

As the residue from a substituted or unsubstituted, mono or polyfunctional alcohol, amine, mercaptan or molecular mixture thereof, $R^4$ can, for example, be any of the following: when s is 1, $R^4$ is selected from 1-morpholinyl, 1-piperidinyl, 1-pyrrolidinyl, $R^7$-Y- in which

Y is -O-, $-N(R^6)-$, -S-, -NH-C(=O)-NH-NH-, -O-C(=O)-NH-NH-, -NH-C(=O)-C(=O)-NH-NH-, and -O-C(=O)-C(=O)-NH-NH-;

$R^6$ is selected from

hydrogen, aliphatic of 1-22 carbons, aryl of 6-10 carbons, araliphatic of 7-22 carbons, and alicyclic of 5-12 carbons which may optionally contain heteroatom ring members selected from nitrogen, oxygen and sulfur; and

$R^7$ is selected from

$R^5$, trialkylsilylalkyl of 5-20 carbons total, alkyldiarylsilyalkyl of 15-20 carbons total, alkoxyalkyl of 3-20 carbons total, 3-(2H-benzotriazol-2-yl)-2-hydroxybenzyl, 4-(2,2-di(methoxycarbonyl)ethenyl]phenyl, /triethoxysilylpropyl, polyalkyl such as $CH_3-(CH_2)_c$-in which c is an integer of 25 to 50;

when s is 1 or more, $R^4$ is selected from the following di-, tri-, or polyvalent groups (1)-(16) with the understanding that any valence not satisified by attachment to the acyl group shown in I is satisfied by a group selected from $R^2$ and $R^5$:

(1) 1,4-piperazindiyl, and

(2) $-Y-R^8-Y-$wherein

Y is as previously defined,

$R^8$ is selected from aliphatic diradical of 2-20 carbons, alicyclic diradical of 5-20 carbons, aryl diradical of 6-10 carbons, araliphatic diradical of 7-22 carbons, alkyldiarylene of 13-20 carbons, cycloalkyldialkyl of 7-12 carbons, 4,4′-oxybis(phenylene)-diyl, 1,4-phenylenebis(dimethylsilyl), 1,2- and 1,4-phenylenebis(oxyalkyl) of 10-14 carbons, 1,2-, 1,3-, and 1,4-phenylenebis(alkyl) of 8-16 carbons, oxybis(dimethylsilylpropyl), 4,4′-biphenyl-diyl, 2,2-propane-bis(phenylene)-p,p′-diyl, diphenylsulfone-4,4′-diyl; poly(alkoxy)dialkyl such as

$$-(CH_2)_d-\underset{R^1}{\underset{|}{CH}}-(CH_2-\underset{R^1}{\underset{|}{CH}}-O)_e-(CH_2)_d-\underset{R^1}{\underset{|}{CH}}-$$

in which d is an integer of 1-2 and e is an integer of 0-350;

polyesters such as

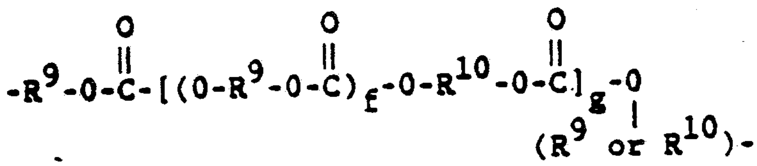

in which f and g are integers independently selected from 1-5, and $R^9$ and $R^{10}$ are independently selected from aliphatic diradical of 2-20 carbons, alicyclic diradical of 5-12 carbons, aryl diradical of 6-10 carbons, araliphatic diradical of 7-22 carbons, alkyldiarylene of 13-20 carbons, and cycloalkyldialkyl of 7-12 carbons; copolyesters such as

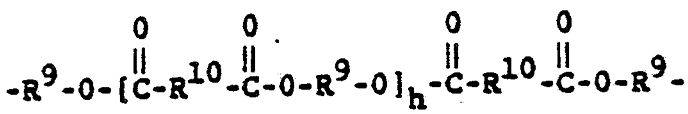

in which h is an integer from 0 to 10; unsaturated polyolefins such as

-[polybutadiene]- in which the polybutadiene has a molecular weight of from 2000 to 3500; poly(mercaptoethers)such as

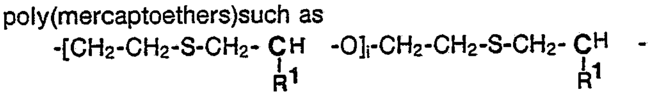

in which i is an integer from 2-12; unsaturated copolymers such as

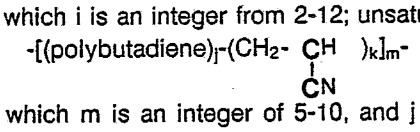

in which m is an integer of 5-10, and j and k are component fractions, k is selected from about 0.1 to 0.3 and j is l-k;

bicyclic divalent hydrocarbons such as

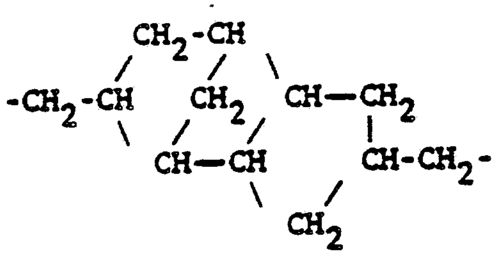

poly(organosiloxanes) such as

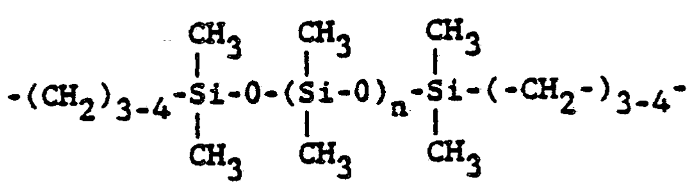

in which n is an integer of 5 to 3000; and

(3) polyester such as

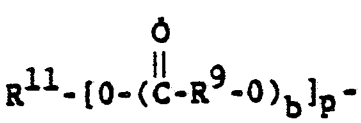

in which b is an integer of 1-10 and may be different for each of the p repeating groups, p is 2 or 3, and when p is 2, $R^{11}$ is an aliphatic diradical of 2 to 4 carbons, and when f is 3, $R^{11}$ is an aliphatic triradical of 3 to 8 carbons; and

(4) 4-aza-1,7-dioxaheptane-1,4,7-triyl, and

(5) 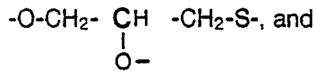, and

(6)

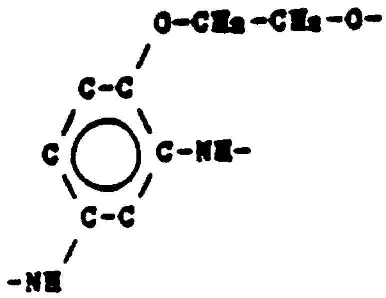

, and

(7)

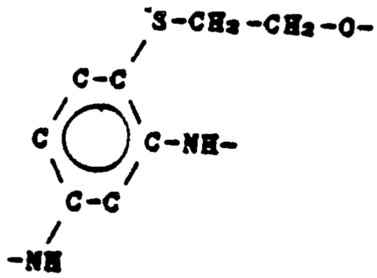

, and

(8)

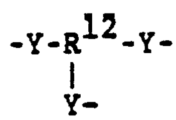

wherein

Y is as previously defined,

$R^{12}$ is chosen from aliphatic triradical of 3 to 20 carbons, aromatic triradical of 6 to 13, 1,3,5-triazine-2,4,6-triyl, triisopropylbenzene-alpha, alpha′, alpha″-triyl, nitrillotriethyl, $-CH_2-CH_2-CH_2-N(CH_2-CH_2-)_2$; and

(9)

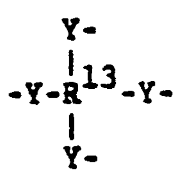

wherein

Y is as previously defined,

$R^{13}$ is an aliphatic tetraradical of 4 to 20 carbons; and

(10)

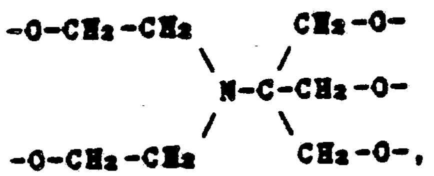
, and

(11)

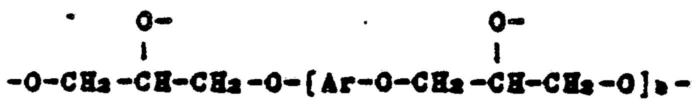
wherein

Ar is 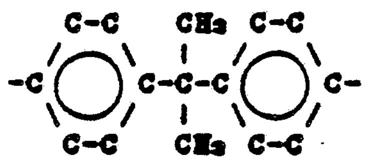

and b is as previously defined; and

(12) $-NH-(CH_2-CH_2-N)_{1-2}-CH_2-CH_2-NH-$ and

(13)

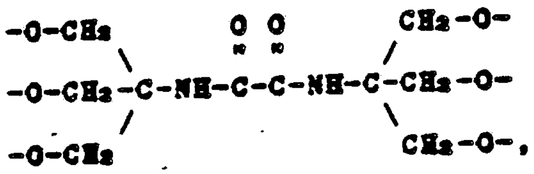
, and

(14)

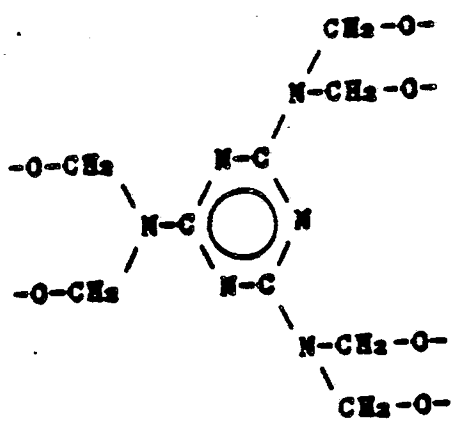
, and

(15)

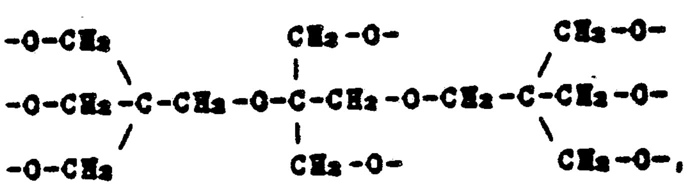
, and

and (16) a polymeric or copolymeric radical containing recurring units

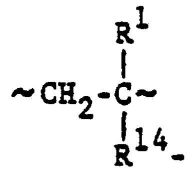

wherein

$R^{14}$ is selected from -O-, -N($R^6$)-, -$CH_2$-O-, -C(=O)-O-$CH_2$-CH(OH)-$CH_2$-O-, -C(=O)-O-$CH_2$-$CH_2$-O-, and -C-(=O)-O-$CH_2$-$CH_2$-$CH_2$-O-, and ~ is the polymer or copolymer backbone in which the units recur.

As the anion from a carboxylic acid, A can be, for example, oxalate, benzoate, formate, acetate, 4-methylbenzoate, isobutyrate, propionate, succinate, or terephthalate.

Substituents for the aliphatic, aromatic, araliphatic and alicyclic radicals include one or more groups chosen from alkyl of 1 to 4 carbons, alkoxy of 1 to 4 carbons, acyl or acyloxy of 1 to 12 carbons, alkoxycarbonyl of 2 to 5 carbons, arylcarbonyl of 7 to 11 carbons, acryloyloxy, methacryloyloxy, aryloxy of 6 to 10 carbons, aralkyl of 7 to 10 carbons, amino, hydroxy, carboxy, nitrile, chloro, bromo, epoxy, alkyl mercapto of 1 to 4 carbons, aryl mercapto of 6 to 10 carbons, alkylamino of 1 to 4 carbons, dialkylamino of 2 to 8 carbons total, arylamino of 6 to 10 carbons, aryl alkyl amino of 7 to 10 carbons, and trialkoxysilyl of 3 to 9 carbons.

The preparation of the compounds of this invention involves the formation of an imide by reaction of trimellitic acid derivatives and an amino-substituted hindered amine light stabilizer of formula

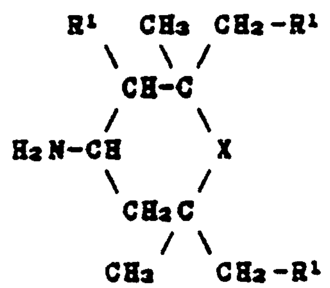

in which $R^1$ and X are as previously described. Syntheses of such hindered amine light stabilizers are well-known in the art.

Trimellitic acid derivatives which can be used are of general structures

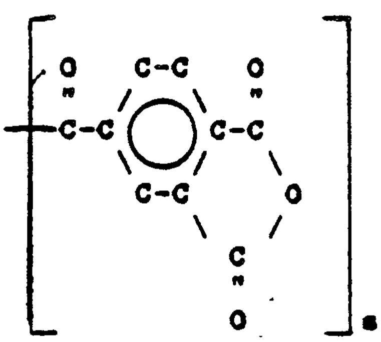

and

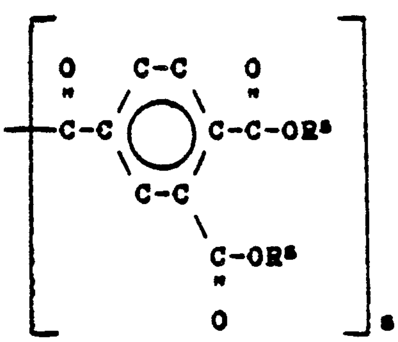

where $R^4$, $R^5$ and s are as previously defined. The imide formed is structure I.

All the compounds of this invention may be considered as derivatives of the following formula which is shown without optional substitution to illustrate the addition product (imide) from two compounds: trimellitic anhydride and 2,2,6,6-tetrasubstituted-4-aminopiperidine derivative shown above:

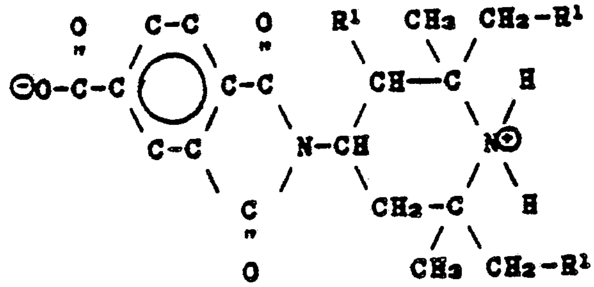

II

This compound which contains both carboxylic acid and amine base exists as an inner salt (imino carboxylate). This is not necessarily true for all possible derivatives, and in more general form the addition product becomes

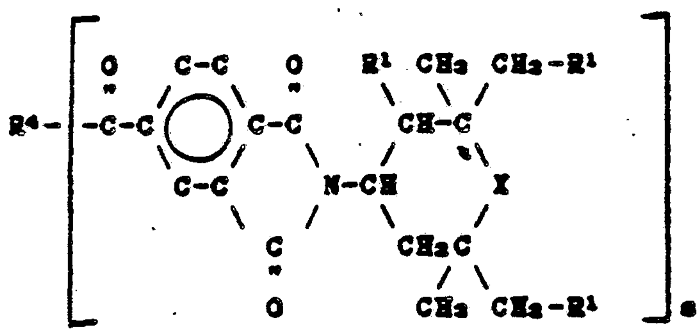

I

wherein $R^1$, $R^4$, s and X are as previously defined.

Depending on the definition of $R^4$, $R^2$, and $R^3$, additional reaction may be necessary to prepare other compounds of this invention. In this case, additional starting materials can be used.

Other starting materials which are commercially available are amines, alcohols, mercaptans, alkyl halides, amides, anhydrides, acyl halides, chloroformates, epoxides, and esters.

Some of the starting materials which can be used in the preparation of this invention are commercially available polymeric materials such as:

1. K-FLEXTM polyester polyols, available from King Industries Specialty Chemicals.
2. FomrezTM 53, a hydroxyl terminated saturated polyester available from Whitco Chemical Organics Division.
3. JeffamineTM polyoxyalkyleneamines, primary amine terminated polyethers available as monoamines, diamines and triamines, available from Texaco Chemical Company.
4. Poly bdTM, liquid, hydroxyl terminated polymers of butadiene, available from ARCO chemical Company.
5. PermapolTM, polythioether diols available from Products Research & Chemical Corporation.
6. PolywaxTM OH alcohols, primary linear polymeric alcohols with a fully saturated hydrocarbon backbone, available from Petrolite Corporation.
7. HycarTM reactive liquid polymers, hydroxyl terminated butadiene/acrylonitrile copolymers available from Goodrich Chemical Company, Chemical Group.
8. RJ-100TM and RJ-101TM, styrene/allyl alcohol copolymers available from Monsanto Company.
9. Saytech Experimental Polyol 42-77, a brominated polyester polyol available from Saytech Inc.
10. MAZERTM SFR Reactive Silicone fluids, which are reactive polydimethylsiloxanes with terminal hydroxy sites, available from MAZER Chemicals, Inc.

11. TONE™ Polyols, diols and triols based on polycaprolactone, available from Union Carbide Corporation.

12. Duracarb™ hydroxy terminated aliphatic polycarbonates, available from PPG Industries.

13. Epoxy Resins such as those derived from Bisphenol-A and epichlorohydrin which have 1 or more residual epoxy groups attached to the polymer.

14. NIAX™ polyether polyols available from Union Carbide Corporation.

15. Carbowax™ polyethylene glycols and methoxy polyethylene glycols available from Union Carbide Corporation.

16. Any of a number of surfactants with a free hydroxy groups such as Triton™ X-100, a product of Rhom & Haas Company.

In addition, there are many other polymeric derivatives that can be made. The HALS moiety can be attached to any polymer with reactive functionality similar to those discussed above. Particularly useful are polymers containing residues of monomers such as vinyl alcohol, 2-hydroxyethyl (meth)acrylate, glycidyl (meth)acrylate, hydroxypropyl (meth)acrylate. Additional commerically available polymers have hydroxy groups both along the polymer chain or as endgroups. Examples of these types of polymers are Bisphenol-A polycarbonate, polyphenylene oxide, hydroxypropyl cellulose, and hydroxypropyl methyl cellulose.

Inner salt II is a versatile intermediate for further elaboration into additional compositions of this invention. In addition to the examples, another method of preparation of salt II would involve the reaction of the amino-substituted piperidine with an appropriate diester, ester-acid, amide-ester or ester-acid chloride or diacid dichloride. Such preparations are described in the chemical literature.

Esterification of II may be accomplished in many ways. Alkylation by a reactive alkyl chloride, bromide or iodide can be done under neutral or alkaline conditions in a solvent such as aromatic hydrocarbons, chlorinated alkanes, ketones (such as acetone, methyl ethyl ketone), ethers (such as methyl t-butyl ether, tetrahydrofuran), amides (such as N-methylpyrrolidone, N,N,-dialkylformamide), sulfolane and dimethylsulfoxide, alone or in combination. Esterification by epoxides is carried out in a similar manner. The concentration of reactants in the solvent is not critical as long as both reactants are at least partially soluble. The preferred solvent is dimethylformamide or solvent mixtures in which it is present. The mole ratio of II to alkylating agent can range from 1:1 to 1:10 and varies based on the desired product. If the hindered amine is not tertiary (X is -NH- or -NH $_2^{\oplus}$-) before this reaction, the alkylation may include both carboxyl and amine substitution. This can be controlled via stoichometry to favor only alkylation of carboxyl (1 equivalent alkylating agent) to alkylation of both carboxyl and amine (2 equivalents or more). The alkylation can be done in the presence of an acid acceptor such as a tertiary amine, or an inorganic base such as an alkali metal carbonate, bicarbonate or hydroxide. The added base is required to liberate the amine if it is participating in a salt. Time and temperature conditions are such that the reaction proceeds at a reasonable rate. Typical conditions would be ambient temperature up to the refluxing temperature of the chosen solvent (up to 200 °C) and a reaction duration of 1 to 72 hours. The preferred temperature range is room temperature up to 150 °C.

Also beneficial in certain instances is the inclusion of a phase transfer catalyst (PTC) to enable the reaction either by increasing its rate or by aiding dissolution of the starting materials. The amount of PTC is generally 0.01 to 0.5 mole% based on II, preferably 0.05 to 0.2 mole%. An example of a PTC would be a tetraalkylammonium halide. Many such compounds are commercially available.

The acid halide of II is a versatile intermediate which is prepared by contacting the free acid or an alkali metal salt with a reactive halogenating agent such as thionyl chloride, thionyl bromide, phosphorus trichloride or tribromide, phosphorus pentachloride or pentabromide, phosphorus oxychloride or other known halogenating agents. This reaction is best done using excess halogenating agent as solvent although inert solvents such as aromatic hydrocarbons or halogenated alkanes may also be used. The formation reaction can be done at a variety of temperatures for various time periods depending upon the reactivity of the halogenating agent and the acid derivative. Temperatures from ambient up to refluxing solvent (up to 150 °C) may be employed. The reaction is continued until conversion is complete, generally 1 to 24 hours or longer.

The product of reaction is the ammonium salt of the acid halide and this often precipitates from the reaction mixture as formed. In this instance, the product is conveniently isolated by filtration from the reaction mixture. If the product does not precipitate, the solvent and excess halogenating agent is stripped away from the product using common methods. This intermediate must be protected from moisture which hydrolyzes it back to the acid.. A preferred method is described in the examples section.

The mono-or poly-ester, thioester and amide derivatives (or mixtures therof) can be prepared via the acid halide intermediate, by reaction with the appropriate mono- or poly-alcohol, amine or mercaptan (or mixture). This acylation reaction is best carried out in solvent with an acid acceptor to remove the halogen

acid as formed and to free the acid halide ammonium salt. An excess of the compound being acylated is desirable, especially from mono-acrylated compositions. In the case of the mono or polyamine starting materials excess amine can serve as the halogen acceptor. In the case of the mono or poly alcohol or mercaptan starting materials an additional base, either a tertiary amine or an inorganic base such as an alkali metal carbonate, bicarbonate or hydroxide can be used. The mole ratio of alcohol, mercaptan and amine groups to the acid halide is calculated based on the equivalents of reactive hydroxyl, mercapto and amino groups available and the desired degree of substitution, and providing one equivalent of acid halide for each equivalent of hydroxyl, mercaptan or amine to be acylated. For a heterogeneous base, a PTC as described above can be beneficial. The acylation reaction may be carried out over a wide range of temperatures, particularly ambient temperature up to the refluxing temperature of the reaction medium (up to about 150 °C). The duration of the reaction is chosen based on product, the rate of conversion of starting material to product, and can vary from 1 to 72 hours.

Polymeric derivatives can be prepared by five main methods: acylation of polymers bearing reactive hydroxyl, amino, mercapto or epoxy groups; (co)polymerization of derivatives of the acid, as for example, 2-(methacryloyloxy)ethyl ester of the allyl ester; graft polymerization of the same monomeric HALS onto a polymer backbone; functionalization of condensation polymers through use of a molecular weight regulator; and attachment of a suitably structured HALS derivative through its use as a chain transfer agent.

The alkylation is conducted as described previously.

The polymerization of monomeric derivatives either alone or with other monomers is performed using any of the well known methods employed in the art for copolymerizing ethylenic or vinyl aromatic monomers with the monomer functionality incorporated into the HALS-imide. Description of such methods are described in Kirk-Othmer Encyclopedia of Chemical Technology, 3rd Ed., Vol 18, "Polymerization Mechanisms and Processes", pp. 720-744. In addition 5 to 40 percent by weight of one of the known elastomers may be incorporated into the copolymer by copolymerizing the monomers in the presence of the rubber. Preferably, the elastomers are incorporated into the monomer mixture prior to polymerization using, for example, the methods of U.S. Patents 4,097,551 or 4,486,570. Preferred rubbers are diene rubbers such as homopolymers of conjugated dienes such as butadiene, isoprene, chloroprene or piperylene and copolymers of such dienes with up to 50 mole percent of one or more copolymerizable mono-ethyleneically unsaturated monomers, such as styrene, substituted styrenes, acrylonitrile, methacrylonitrile and isobutylene.

The polymeric derivatives can also be prepared by grafting the monomeric HALS-imide onto a polymer. The polymer may be high or low density polyethylene, a polypropylene or a copolymer of alpha olefins having up to about six carbons, or any other polymer having a labile hydrogen on the polymer backbone. Examples of such copolymers are ethylene-butene-1, ethylene-propylene and propylene-butene-1 copolymers. The method of grafting the HALS monomer onto the polymers is similar to the methods known in the art for such reactions. Briefly, the preparation consists of treating the polymer with a free radical initiator which generates free radicals on the polymer. The free radical sites on the polymer can then add on the unsaturated HALS-monomer. Active radical sites on the polymer backbone can also be induced by subjecting the polymer to the action of high energy ionizing radiation such as gamma rays, X-rays or high speed electrons or by simply milling the polymer in the presence of air. Examples of applicable methods are described in U.S. Patents 3,483,276 and 4,506,056.

The free acid group of the HALS derivative can be used to control the molecular weight of the polymers prepared by condensation polymerization, such as polyamides, polycarbonates and polyesters. The technique of adding a monobasic acid for molecular weight control is well known in the art. In this process, the monobasic acid (in this case the HALS derivative) becomes attached to the polymer molecule as an endgroup.

A HALS derivative with a labile hydrogen can be introduced into a polymerization mixture as a chain transfer agent. In this capacity, the HALS would also be incorporated as a polymer endgroup. The mechanism here consists of termination of a growing polymer chain by donation of the labile hydrogen and initiation of another polymer chain from the active site thus formed on the HALS molecule. Such a technique is known in the art.

In the following specific illustrative examples the N-(hindered amine)-phthalimide group appears as both a substituent and as a parent group. The substituent nomenclature used is N-(hindered amine)phthalimide-4-carbonyl-. When cited as a molecular parent, it follows accepted convention.

1. N-(2,2,6,6-tetramethyl-4-piperidinyl)-4-(methoxycarbonyl)phthalimide
2. N-(2,2,6,6-tetramethyl-4-piperidinyl)-4-(bromocarbonyl)phthalimide, hydrobromide salt
3. N-(2,2,6,6-tetramethyl-4-piperidinyl)-4-(carboxy)phthalimide, p-toluenesulfonate salt
4. N-(2,2,6,6-tetramethyl-4-piperidinyl)-4-((3,5-di-t-butyl-4-hydroxybenzyloxy)carbonyl)phthalimide

5. N-(2,2,6,6-tetramethyl-4-piperidinyl)-4-{[2-hydroxy-3-(2H-benzotriazol-2-yl)-5-methylbenzyl]-aminocarbonyl}phthalimide

6. N-(2,2,6,6-tetramethyl-4-piperidinyl)-4-[5-(4-benzoyl-3-hydroxyphenoxy)-1,4-dioxo-2,3-diazapentyl]-phthalimide

7. N-[1-(2,3-epoxy)propyl-2,2,6,6-tetramethyl-4-piperidinyl]-4-(methoxycarbonyl)phthalimide

8. N-[1-(2,3-epoxy)propyl-2,2,6,6-tetramethyl-4-piperidinyl]-4-[2,3-(epoxy)propoxycarbonyl]phthalimide

9. 4,4′-(4,13-dihydroxy-2,6,11,15-tetraoxa-1,16-dioxohexadecane-1,16-diyl)-bis[N-(2,2,6,6-tetramethyl-4-piperidinyl)phthalimide]

10. N-(2,2,6,6-tetramethyl-4-piperidinyl)-4-{[3-(methacryloxy)-2-hydroxypropoxy]carbonyl}phthalimide

11. N-(2,2,6,6-tetramethyl-4-piperidinyl)-4-{[3-(allyloxy)-2-hydroxypropoxy]carbonyl}phthalimide

12. N-(2,2,6,6-tetramethyl-4-piperidinyl)-4-{[3-(n-butoxy)-2-hydroxypropoxy]carbonyl}phthalimide

13. N-(2,2,6,6-tetramethyl-4-piperidinyl)-4-{[2-hydroxy-4-vinylcyclohexyloxy]carbonyl}phthalimide

14. N-(2,2,6,6-tetramethyl-4-piperidinyl)-4-{[2-phenyl-2-hydroxyethoxy]carbonyl}phthalimide

15. N-(2,2,6,6-tetramethyl-4-piperidinyl)-4-{[2-hydroxy-2-methyl-5-(isopropylidene)cyclohexyloxy]-carbonyl}phthalimide

16. N-(2,2,6,6-tetramethyl-4-piperidinyl)-4- {[3-phenoxy-2-hydroxypropoxy]carbonyl}phthalimide

17. N-(2,2,6,6-tetramethyl-4-piperidinyl)-4-{[3-(4-butylphenoxy)-2-hydroxypropoxy]-carbonyl}phthalimide

18. N-(2,2,6,6-tetramethyl-4-piperidinyl)-4-{[1,5,5-trimethyl-1-hydroxybicyclo(3.1.1)heptane-2-yloxy]-carbonyl}phthalimide

19. N-(2,2,6,6-tetramethyl-4-piperidinyl)-4- {[3-(3-pentadecylphenoxy)-2-hydroxypropoxy]-carbonyl}phthalimide

20. N-(2,2,6,6-tetramethyl-4-piperidinyl)-4- {[3-(t-alkyl($C_8$-$C_{10}$)carbonyloxy)-2-hydroxypropoxy]-carbonyl}phthalimide

21. 4,4′-(4,8-dihydroxy-2,6,10-trioxa-1,11-dioxoundecane-1,11-diyl)-bis[N-2,2,6,6-tetramethyl-4-piperidinyl)phthalimide]

22. 1,2,-di{2-hydroxy-4-oxa-4-[N-(2,2,6,6-tetramethyl-4-piperidinyl)phthalimide-4-carbonyl]-butyl}benzene

23. 1,2-di{2-hydroxy-4-oxa-4-[N-(2,2,6,6-tetramethyl-4-piperidinyl)phthalimide-4-carbonyl]-butoxy}benzene

24. 4,4′-(4,14-dihydroxy-2,6,9,12,16-pentaoxa-1,17-dioxoheptadecane-1,17-diyl)-bis[N(2,2,6,6-tetramethyl-4-piperidinyl)phthalimide]

25. 4,4′-(4,5-dihydroxy-2,7-dioxa-1,8-dioxoctane-1,8-diyl)-bis[N-(2,2,6,6-tetramethyl-4-piperidinyl)-phthalimide]

26. 4,4′-(4,6-dihydroxy-4,6-dimethyl-2,8-dioxa- 1,9-dioxononane-1,9-diyl)-bis[N-2,2,6,6-tetramethyl-4-piperidinyl)phthalimide]

27. N-(2,2,6,6-tetramethyl-4-piperidinyl)-4-{[(3,5-di(n-butylmercapto)-2,4,6-triazin-1-yl]-aminocarbonyl}phthalimide

28 N-(2,2,6,6-tetramethyl-4-piperidinyl)-4-{[3,5-diamino-2,4,6-triazin-1-yl]minocarbonyl}phthalimide

29. N-(2,2,6,6-tetramethyl-4-piperidinyl)-4-{[3,5-dimethoxy-2,4,6-triazin-1-yl]-aminocarbonyl}phthalimide

30. N-(2,2,6,6-tetramethyl-4-piperidinyl)-4-[(2-bromoethoxy)carbonyl]phthalimide

31. 6-(n-butylmercapto)-2,4-di[N-(2,2,6,6-tetramethyl-4-piperidinyl)phthalimide-4-carbonylamino]-1,3,5-triazine

32. N-(2,2,6,6-tetramethyl-4-piperidinyl)[2-(n-butoxy)ethoxycarbonyl]phthalimide

33. N-(2,2,6,6-tetramethyl-4-piperidinyl)[1-methyl-2-(ethoxycarbonyl)ethoxycarbonyl]phthalimide

34. N-(2,2,6,6-tetramethyl-4-piperidinyl)-4-[6-(2,2,6,6-tetramethyl-4-peridinyl)-2,3,6-(triaza)-1,4,5-(trioxo)hexyl]phthalimide

35. N-(2,2,6,6-tetramethyl-4-piperidinyl)-4-[6- (3,5-di-t-butyl-4-hydroxyphenyl)-2,3,-(diaza)-1,4-(dioxo)-hexyl]phthalimide

36. N-(2,2,6,6-tetramethyl-4-piperidinyl)-4-[6-(n-hexylmercapto)-2,3-(diaza)-1,4-(dioxo)hexyl]-phthalimide

37. N-(2,2,6,6-tetramethyl-4-piperidinyl)-4-[7-(2,2,6,6-tetramethyl-4-piperidinyl)-2,3,7-(triaza)-1,4-(dioxo)heptyl]phthalimide

38. 1,4-di[N-(2,2,6,6-tetramethyl-4-piperidinyl)phthalimide-4-carbonyl]piperazine

39. 1,4-di[N-(2,2,6,6-tetramethyl-4-piperidinyl)phthalimide-4-carbonyloxy]benzene

40. N-{1-[(butoxycarbonyl)methyl]-2,2,6,6-tetramethyl-4-piperidinyl}-4-(butoxycarbonyl)phthalimide

41. 2,2-di{4-[N-(2,2,6,6-tetramethyl-4-piperidinyl)phthalimide-4-carbonyloxy]phenyl}propane

42. 4,4′-(4-hydroxy-2,6-dioxa-1,7-dioxoheptane-1,7-diyl)-bis[N-(2,2,6,6-tetramethyl-4-piperidinyl)-phthalimide]

43. 4,4′-(2,5,8-trioxa-1,9-dioxononane-1,9-diyl)bis[N- (2,2,6,6-tetramethyl-4-piperidinyl)phthalimide]

44. N-(2,2,6,6-tetramethyl-4-piperidinyl)-4-{[3-(trimethoxysilyl)propyl]aminocarbonyl}phthalimide

45. N-(1-benzyl-2,2,6,6-tetramethyl-4-piperidinyl)- 4-{[2-(dimethylamino)ethyl]-aminocarbonyl}phthalimide

46. 1,4-di[N-(2,2,6,6-tetramethyl-4-piperidinyl)phthalimide-4-carbonylamino[benzene

47. 4,4′-[4,4-di(hydroxymethyl)-2,6-dioxa-1,7-dioxoheptane-1,7-diyl)]-bis[N-(2,2,6,6-tetramethyl-4-piperidinyl)phthalimide]

48. 4,4′-{4,4-di{[N-(2,2,6,6-tetramethyl-4-piperidinyl)phthalimide-4-carbonyloxy]methyl}-2,6-dioxa-1,7-dioxoheptane-1,7-diyl}bis[N-2,2,6,6-tetramethyl-4-piperidinyl)phthalimide]

49. N-(2,2,6,6-tetramethyl-4-piperidinyl)-4-carboxyphthalimide, ester with poly(caprolactone)-triol, 3:1

50. N-(2,2,6,6-tetramethyl-4-piperidinyl)-4-carboxyphthalimide, ester with poly(propyleneoxide)-diol, 2:1

51. N-(2,2,6,6-tetramethyl-4-piperidinyl) 4-carboxyphthalimide, product with epoxy resin (Bisphenol-A/epichlorohydrin)

52. 1,4-di{[N-(2,2,6,6-tetramethyl-4-piperidinyl)phthalimide-4-carbonyloxy]methyl)cyclohexane

53. N-(2,2,6,6-tetramethyl-4-piperidinyl)-4-{[2-(4-benzoyl-3-hydroxyphenoxy)ethoxy}carbonyl] phthalimide

54. 7-[N-(2,2,6,6-tetramethyl-4-piperidinyl)phthalimide-4-carbonyloxy]3-{[N-(2,2,6,6-tetramethyl-4-piperidinyl)phthalimide-4-carbonyloxy]methyl}tricyclo(5.2.0$^{1,5}$)decane

55. 4,4′-{4-[N-(2,2,6,6-tetramethyl-4-piperidinyl)phthalimide-4-carbonylmercapto]-2,6-dioxa-1,7-dioxoheptane-1,7-diyl}-bis[N-(2,2,6,6-tetramethyl-4-piperidinyl)phthalimide]

56. 1,3-di[N-(2,2,6,6-tetramethyl-4-piperidinyl)phthalimide-4-carbonylamino]-4-{6-[N-(2,2,6,6-tetramethyl-4-piperidinyl)phthalimide-4-carbonyl]-5-oxa-1-thiapentyl}benzene

57. N-(1,2,2,6,6-pentamethyl-4-piperidinyl)-4-{[(3-benzoyloxy)phenoxy]carbonyl}phthalimide

58. N-[1-(2-hydroxyethyl)-2,2,6,6-tetramethyl-4-piperidinyl]-4-{[2-(acryloyloxy)ethoxy]-carbonylphthalimide

59. N-(2,2,6,6-tetramethyl-4-piperidinyl)-4-[(2-aminoethyl)aminocarbonyl]phthalimide

60. methyl methacrylate/glycidyl methacrylate/N-(2,2,6,6-tetramethyl-4-piperidinyl)-4-{[3-(methacryloyloxy)-2-hydroxypropoxy]carbonyl}phthalimide terpolymer

61. N-(2,2,6,6-tetramethyl-4-piperidinyl)-4-carboxy phthalimide, ester with poly[oxycarbonyloxy-1,4-phenylene(1-methylethylidene)-1,4-phenylene], 2:1

62. N-(2,2,6,6-tetramethyl-4-piperidinyl)-4-{[(2-(3,3,-diphenyl-2-cyanoacryloyloxy)ethoxy]carbonyl)-phthalimide

63. N-(2,2,6,6-tetramethyl-4-piperidinyl)-4-(2-hydroxypropoxycarbonyl)phthalimide

64. N-(2,2,6,6-tetramethyl-4-piperidinyl)-4-{[2-(3,3,-diphenyl-2-cyanoacryloyloxy)ethoxyl]-carbonyl}phthalimide

65. N-(2,2,6,6-tetramethyl-4-piperidinyl)-4-(2-hydroxypropoxycarbonyl)phthalimide

66. 2-{N-[methoxymethyl]-N-[N-(2,2,6,6-tetramethyl-4-piperidinyl)phthalimide-4-carbonyloxymethyl]-amino}-4,6-di[N,N-di(methoxymethyl)amino]-1,3,5-triazine

The HALS of this invention can be used to protect polymeric materials from photooxidative degradation. The HALS may be incorporated into the polymer at any effective concentration, generally 0.01 to 5% by weight based on the weight of polymer to be stabilized, preferably 0.05 to 2% by weight. In most cases 0.1 to about 1% by weight of the final polymer composition is sufficient. Examples of polymers and copolymers which may be stabilized by these compounds include:

1. Polyolefins such as high, low and linear low density polyethylenes, which may be optionally crosslinked, polypropylene, polyisobutylene, poly(methylbutene-1), polyacetylene and in general polyolefins derived from monomers having from two to about ten carbon atoms and mixtures thereof.

2. Polyolefins derived from diolefins such as polybutadiene and polyisoprene.

3. Copolymers of mono or diolefins such as ethylene-propylene, propylene-butene-1, propylene-isobutylene and ethylene-butene-1 copolymer.

4. Terpolymers of ethylene and propylene with dienes (EPDM) such as butadiene, hexadiene, dicyclopentadiene and ethylidene norbornene.

5. Copolymers of alpha-olefins with acrylic acid or methacrylic acids or their derivatives such as ethylene-acrylic acid, ethylene-methacrylic acid and ethylene-ethyl acrylate copolymers.

6. Styrenic polymers such as polystyrene (PS) and poly(p-methylstyrene).

7. Styrenic copolymers and terpolymers such as styrene-butadiene (SBR), styrene-allyl alcohol and styrene-acrylonitrile (SAN), styrene-acrylonitrile-methacrylate terpolymer, styrene-butadiene-styrene block copolymers (SBS), rubber modified styrenics such as styrene-acrylonitrile copolymers modified with acrylic ester polymer (ASA), graft copolymers of styrene on rubbers such a polybutadiene (HIPS), polyisoprene or styrene-butadiene-styrene block copolymers, graft copolymers of styrene-acrylonitrile on rubbers such as butadiene (ABS), polyisoprene or styrene-butadiene-styrene block copolymers, graft copolymers of styrene-methyl methacrylate on rubbers such as polybutadiene (MBS), butadiene-styrene radial block copolymers (e.g. KRO 3 of Phillips Petroleum Co.), selectively hydrogenated butadiene-styrene block copolymers (e.g. Kraton G from Shell Chemical Co.) and mixtures thereof.

8. Polymers and copolymers derived from halogen-containing vinyl monomers such as poly(vinyl chloride), poly(vinyl fluoride), poly(vinylidene chloride), poly(vinylidene fluoride), poly(tetrafluoroethylene) (PTFE), vinyl chloride-vinyl acetate copolymers, vinylidene chloride-vinyl acetate copolymers and ethylene-tetrafluoroethylene copolymers.

9. Halogenated rubbers such as chlorinated and/or brominated butyl rubbers such as chlorinated and fluoroelastomers.

10. Polymers and copolymers derived from alpha, beta-unsaturated acids, anhydrides, ester, amides and nitriles or combinations thereof such as polymers or copolymers of acrylic and methacrylic acids, alkyl and/or glycidyl acrylates and methacrylates, acrylamide and methacrylamide, acrylonitrile, maleic anhydride, maleimide, the various anhydride containing polymers and copolymers described in this disclosure, copolymers of the above polymers and various blends and mixtures thereof as well as rubber modified versions of the above polymers and copolymers.

11. Polymers and copolymers derived from unsaturated alcohols or their acylated derivatives such as poly(vinyl alcohol), poly(vinyl acetate), poly(vinyl stearate), poly(vinyl benzoate), poly(vinyl maleate), poly-(vinyl butyral), poly(allyl phthalate), poly(allyl diethylene glycol carbonate) (ADC), ethylene-vinyl acetate copolymer and ethylene-vinyl alcohol copolymers.

12. Polymers and copolymers derived from unsaturated amines such as poly(allyl melamine).

13. Polymers and copolymers derived from epoxides such as polyethylene oxide, polypropylene oxide and copolymers thereof as well as polymers derived from bis-glycidyl ethers.

14. Poly(phenylene oxides), poly(phenylene ethers) and modifications thereof containing grafted polystyrene or rubbers as well as their various blends with polystyrene, rubber modified polystyrenes or nylon.

15. Polycarbonates and especially the aromatic polycarbonates such as those derived from phosgene and bisphenols such as bisphenol-A, tetrabromobisphenol-A and tetramethylbisphenol-A.

16. Polyester derived from dicarboxylic acids and diols and/or hydroxycarboxylic acids or their corresponding lactones such as polyalkylene phthalates (e.g., polyethylene terephthalate (PET), polybutylene terephthalate (PBT), and poly(1,4-dimethylcyclohexane terephthalate) or copolymers thereof) and polylactones such as polycaprolactone.

17. Polyarylates derived from bisphenols (e.g., bisphenol-A) and various aromatic acids such as isophthalic and terephthalic acids or mixtures thereof.

18. Aromatic copolyestercarbonates having carbonate as well as ester linkages present in the backbone of the polymers such as those derived from bisphenols, iso- and terephthaloyl chlorides and phosgene.

19. Polyurethanes and polyureas.

20. Polyacetals such as polyoxymethylenes and polyoxmethylenes which contain ethylene oxide as a comonomer.

21. Polysulfones, polyethersulfones and polyimidesulfones.

22. Polyamides and copolyamides which are derived from diamines and dicarboxylic acids and/or from aminocarboxylic acids or the corresponding lactones such as the following nylons: 6, 6/6, 6/10, 11 and 12.

23. Polyimides, polyetherimides, polyamideimides and copolyetheresters.

24. Crosslinked polymers which are derived from aldehydes on the one hand and from phenols, ureas and melamine on the other hand such as phenol-formaldehyde, urea-formaldehyde and melamine-formaldehyde resins.

25. Alkyl resins such as glycerol-phthalic acid resins and mixtures thereof with melamine-formaldehyde resins.

26. Unsaturated polyester resins which are derived from copolyesters of saturated and unsaturated dicarboxylic acids with polyhydric alcohols as well as from vinyl compounds (crosslinking agents) and also halogen-containing, flame resistant modifications thereof.

27. Natural polymers such as cellulose, natural rubber as well as the chemically modified homologous derivatives thereof such as cellulose acetates, cellulose propionate, cellulose butyrate and the cellulose ethers such as methyl and ethyl cellulose.

In addition the stabilizers of this invention may be used to stabilize various combinations or blends of the above polymers or copolymers. They are particularly useful in the stabilization of polyolefins, acrylic coatings, styrenics, rubber modified styrenics, poly(phenylene oxides) and their various blends with styrenics, rubber-modified styrenics or nylon.

The hindered amine light stabilizers of this invention can be used together with other additives to further enhance the properties of the finished polymer. Examples of other additives that can be used in conjunction with the stabilizers of this invention include other antioxidants such as alkylated monophenols, alkylated hydroquinones, hydroxylated thiodiphenyl ethers, alkylidene-bis-phenols, hindered phenolic benzyl compounds, acylamino-phenols, esters of 2-(3,5-di-t-butyl-4-hydroxyphenyl)propionic acid, esters of 2-(5-t-butyl-4-hydroxy-3-methylphenyl)propionic acid, 2-(3,5-di-t-butyl-4-hydroxyphenyl)propionic acid amides; other UV absorbers and light stabilizers such as 2-(2′-hydroxyphenyl)-2H-benzotriazoles, 2-hydroxybenzophenones, benzylidene malonate esters, esters of substituted or unsubstituted benzoic acids, diphenyl acrylates, nickel chelates, oxalic acid diamides, other hindered amine light stabilizers; other additives such as metal deactivators, phosphites and phosphonites, peroxide decomposers, fillers and reinforcing agents, plasticizers, lubricants, corrosion and rust inhibitors, emulsifiers, mold release agents, carbon black, pigments, fluorescent brighteners, both organic and inorganic flame retardants and nondripping agents, melt flow improvers and antistatic agents. Numerous examples of suitable additives of the above type are given in Canadian Patent 1,190,038.

The polymeric derivatives are particularly attractive, offering enhanced compatability and non-fugitivity when used to stabilize polymer compositions in which they are incorporated.

In the following examples where appropriate, analytical data are included to further define the product. All melting points are uncorrected. Spectral data were obtained using common practices. All NMR spectra were recorded in chloroform-d (unless otherwise specified) relative to tetramethylsilane (0.0 ppm).

## Example 1

### Preparation of N-(2,2,6,6-tetramethyl-4-piperidinyl)-4-carboxyphthalimide, internal salt

Into a 2 liter round bottom flask equipped with a mechanical stirrer, nitrogen atmosphere and thermometer were placed 149.5g (0.75 mol) trimellitic anhydride and 500 ml of acetic acid. This mixture was cooled and stirred while 34.1g (0.85 mol) sodium hydroxide (solid) was added (exothermic). After addition of base, cooling was discontinued and the mixture was stirred while 128.9g (0.82 mol) of 4-amino-2,2,6,6-tetramethylpiperidine was slowly added, using an additional 250 ml of acetic acid to rinse the amine into the flask. This addition was also exothermic, and the heat evolved was used to warm the reaction mixture to about 90°C. After complete addition, the reaction was refluxed for 2 hours. The hot reaction mixture was poured onto a 1000g of ice in a beaker and the mixture was stirred until the ice melted. The solid product was isolated by filtration and slurried twice with 700 ml portions of acetone. A significant static charge complicates handling of the product. The white product weighed 216.2g (87.3% theoretical). The infrared spectrum (KBr pellet) of this compound showed the carbonyl absorptions for the imide (1700 $cm^{-1}$) and the carboxylate (1620 $cm^{-1}$).

The piperidinium salt N-H was indicated by a broad band of 2000-3000 $cm^{-1}$. During heating at 20°C/minute in the differential scanning calorimeter, the salt began decomposing at about 350°C.

## EXAMPLE 2

Preparation of N-(2,2,6,6-tetramethyl-4-piperidinyl)-4-(chlorocarbonyl)phthalimide, hydrochloride salt

Into a dry 500 ml round bottom flask equipped with a magnetic stirrer, reflux condenser and nitrogen atmosphere was placed 175 g (1.5 mol) thionyl chloride. To this was added 69.9 g (0.21 mol) N-(2,2,6,6-tetramethyl-4-piperidinyl)-4-carboxyphthalimide in small portions over a 45 minute period. The resulting mixture was refluxed for an hour before stirring became difficult (due to precipitated product) and 25g (0.21 mol) thionyl chloride was added to alleviate this problem. The mixture was refluxed for three additional hours. The reaction mixture was cooled to room temperature and diluted with 600 ml methyl t-butyl ether. The solid product was isolated by filtration and washed on the funnel with three 100 ml portions of ether. Final removal of solvent was accomplished under high vacuum giving 76.7 g white crystals. By analysis for hydrolyzable chloride, the sample was determined to be 99+% assay. The yield was 94.8%. The infrared spectrum (KBr pellet) of this compound showed the carbonyl absorptions for the imide (1705 $cm^{-1}$) and the carbonyl chloride (1750 $cm^{-1}$).

## EXAMPLE 3

Preparation of N-(2,2,6,6-tetramethyl-4-piperidinyl)-4-(aminocarbonyl)phthalimide

Into a 125 ml Erlenmeyer flask equipped with a thermometer was placed 50 ml of concentrated aqueous ammonium hydroxide. The acid chloride of Example 2 (5.0g., 0.013 mol) was added and the mixture stirred for 30 minutes. The solid product was collected by filtration and washed with several portions of water. The product was dried briefly on the funnel then dissolved in hot methanol and filtered (hot). Upon cooling the product crystallized. The crystallized material was isolated by filtration yielding 1.1g of white crystals (melting 189-200°C). Reduction of the crystallization solvent produced a second crop of product, 0.25g (melting 189-200°C). The infrared spectrum (KBr pellet) of the product showed an intense broad carbonyl for both the amide and the imide at 1680-1720 $cm^{-1}$.

## EXAMPLE 4

Preparation of N-(2,2,6,6-tetramethyl-4-piperidinyl)-4-(ethoxycarbonyl)phthalimide

Into a 125 ml reaction flask equipped with magnetic stirrer, thermometer, additional funnel and nitrogen atmosphere were placed 5.0g (0.013 mol) of acid chloride from Example 2 and 25 ml of ethanol. The additional funnel was charged with 15 ml ethanol and 2.8 g (0.028 mol) of triethylamine. The reaction mixture was stirred as the base was added over a 5 minute period accompanied by a 10°C exotherm. The mixture was stirred at ambient temperature for 2 hours during which time suspended solid slowly dissolved. The reaction mixture was stripped of solvent using an aspirator vacuum. The solid was taken up in 100 ml of toluene in a separatory funnel and washed with 50 ml of saturated sodium bicarbonate and three 50 ml portions of water. The toluene solution was dried with anhydrous magnesium sulfate and stripped of solvent to yield 4.0g of product. This material was recrystallized from 95% ethanol producing 2.6g of white crystals (melting 115-121°C). The infrared spectrum (KBr pellet) of the product showed a doublet carbonyl with absorbance maxima of 1705 $cm^{-1}$ (imide) and 1710 $cm^{-1}$ (ester). The NMR spectrum indicated the presence of the ethyl group (4.4 ppm, quartet, 2H; 1.4 ppm, triplet) and the aromatic ring (7.8-8.0 ppm, doublet, 1H; 8.3-8.5, multiplet, 2H) and the HALS group [4.5-4.8 ppm, multiplet, 1H; 1.0-2.3 ppm several multiplets including two singlets (1.2 and 1.3 ppm)].

EXAMPLE 5

N-(2,2,6,6-tetramethyl-4-piperidinyl)-4-(2,2,6,6-tetramethyl-4-piperidinyloxycarbonyl)phthalimide

Into a 125 ml reaction flask equipped with a magnetic stirrer, thermometer, condenser and nitrogen atmosphere were placed 5.0g (0.013 mol) of the acid chloride of Example 2 and 50 ml of 2% dimethylformamide in methylene chloride. This mixture was stirred druing the addition of 2.1g (0.013 mol) 2,2,6,6-tetramethyl-4-piperidinol and 2.8g (0.028 mol) triethylamine. The reaction was then refluxed for 3 hours. The mixture was cooled and transferred to a separatory funnel with 100 ml of methylene chloride, 25 ml of water and 50 ml of 5% sodium hydroxide. The mixture was shaken, allowed to separate and the organic phase was drawn off. The aqueous phase (and interfacial solid) was extracted with an additional 25 ml of solvent. The combined organic solutions were dried with anhydrous magnesium sulfate and the solvent stripped using aspirator vacuum. The product was 4.1g of slightly yellow crystals (melting 196-200 °C). The infrared spectrum (chloroform) showed the carbonyl absorption at 1705 $cm^{-1}$ (imide and ester). The NMR spectrum showed the anticipated aromatic ring (7.7-7.9 ppm, doublet, 1H; 8.3-8.5, multiplet, 2H) and two HALS groups [5.2-5.7 ppm, multiplet, 1H; 4.4-4.9 ppm, multiplet, 1H; 1.0-2.3 ppm, several multiplets including two singlets (1.2 and 1.3 ppm)]. The UV spectrum showed an absorbance maximum (THF) at 296, molar absorptivity 300.

EXAMPLE 6

Preparation of N-(2,2,6,6-tetramethyl-4-piperidinyl)-4-((2-ethylhexyloxy)carbonyl)phthalimide

Into a 125 ml reaction flask equipped with a magnetic stirrer, thermometer, condenser and nitrogen atmosphere were placed 5.0g (0.013 mol) of the acid chloride of Example 2, 1.7g (0.013 mol) 2-ethylhexanol and 50 ml of 2% dimethylformamide in methylene chloride. This mixture was stirred during the addition of 2.8g (0.028 mol) of triethylamine in 5 ml of methylene chloride. This addition produced an exotherm of about 10 °C. The reaction was then refluxed for 1 hour. The mixture was cooled and transferred to a separatory funnel with 100 ml of methylene chloride and 250 ml of water. The ensuing emulsion was filtered and put back in the funnel and the phases were separated. The organic phase was extracted with 50 ml water and 50 ml of 5% sodium bicarbonate. The organic solution was dried with anhydrous magnesium sulfate and the solvent stripped using an aspirator and high vacuum. Liquid chromatographic analysis of the white solid product indicated the presence of 2 components. The material was redissolved in methylene chloride and extracted with two 50 ml portions of 5% sodium hydroxide. The organic solution was again dried and stripped and the residue recrystallized from 80/20 ethanol/water. After isolation and drying, 1.5g of slightly yellow product was obtained (melting 129-131 °C). The infrared spectrum (KBr pellet) showed the carbonyl absorption at 1720 $cm^{-1}$ (imide and ester). The NMR spectrum showed the anticipated aromatic ring (7.7-7.9 ppm, doublet, 1H 8.2-8.5, multiplet, 2H), and the 2-ethylhexyl and HALS groups [4.4-4.9 ppm, multiplet, 1H; 4.2-4.3 ppm, doublet, 1H; 0.7-2.3 ppm, several multiplets including two singlets (1.2 and 1.3 ppm)].

EXAMPLE 7

Preparation of N-(2,2,6,6-tetramethyl-4-piperidinyl)-4-(dodecylaminocarbonyl)phthalimide

Into a 125 ml reaction flask equipped with a magnetic stirrer, thermometer, condenser and nitrogen atmosphere were placed 2.4g (0.013 mol) of dodecylamine and 40 ml of 2% dimethylformamide in methylene chloride. This mixture was stirred during the addition of 5.0g (0.013 mol) of the acid chloride of Example 2 accompanied by an 8°C exotherm. The reaction mixture was cooled to ambient temperature and 2.8g (0.028 mol) triethylamine was added, producing an exotherm of about 10°C. The reaction was allowed to stir at room temperature for 30 minutes then was transferred to a separatory funnel with 50 ml of methylene chloride and 50 ml water. The phases were separated and the organic phase was further extracted with three 50 ml portions of water. Additional solvent, 50 ml. was added and the organic solution washed with two 50 ml portions of 5% sodium hydroxide. The organic solution was dried with anhydrous magnesium sulfate and the solvent stripped using aspirator and high vacuum systems. The product was 6.1g (98% of theory) of a yellow semi-solid which upon standing became a solid (melting 42-45°C). The infrared spectrum (chloroform) showed two carbonyl absorptions at 1720 $cm^{-1}$ (imide) and 1660 $cm^{-1}$ (amide). The NMR spectrum showed the anticipated aromatic ring (7.7-7.9 ppm, doublet, 1H; 8.1-8.3, multiplet, 2H), the amide N-H (6.4-6.8 ppm, multiplet, 1H), the amide methylene (3.2-3.7 ppm, multiplet, 2H), and HALS groups and amide residue [4.4-4.9 ppm, multiplet, 1H; 0.7-2.3 ppm, several multiplets].

## EXAMPLE 8

Preparation of N-(2,2,6,6-tetramethyl-4-piperidinyl)-4-(dibutylaminocarbonyl)phthalimide

Into a 125 ml reaction flask equipped with a magnetic stirrer, themometer, condenser and nitrogen atmosphere were placed 5.0g (0.013 mol) of acid chloride of Example 2 and 50 ml of 2% dimethylformamide in methylene chloride. This mixture was stirred during the addition of 1.7g (.013 mol) of di-n-butylamine accompanied by a 10°C exotherm. The reaction mixture was cooled to ambient temperature and 2.8g (0.028 mol) triethylamine was added, producing an exotherm of about 10°C. The reaction was cooled to room temperature and allowed to stir for 1 hour then transferred to a separatory funnel with 50 ml of methylene chloride and 50 ml water. Upon shaking, an emulsion formed which was broken by the addition of 25 ml of 5% sodium hydroxide. The phases were separated and the organic phase was further extracted with three 50 ml portions of 5% sodium hydroxide and three 50 ml portions of water. The organic solution was dried with anhydrous magnesium sulfate and the solvent stripped using aspirator and high vacuum systems. The product was 5.0g of a yellow liquid. The infrared spectrum (between salt plates) showed two carbonyl absorptions at 1718 $cm^{-1}$ (imide) and 1642 $cm^{-1}$ (amide). The NMR spectrum showed the anticipated aromatic ring (7.5-8.0 ppm, multiplet, 3H), the amide methylene hydrogens (2.9-3.8 ppm, broad doublet, 4H) and the HALS group [4.4-4.9 ppm, multiplet, 1H; 0.5-2.3 ppm, several multiplets including two singlets (1.2 and 1.3 ppm)].

## EXAMPLE 9

Preparation of N-(2,2,6,6-tetramethyl-4-piperidinyl)-4-(2,2,6,6-tetramethyl-4-piperidinylaminocarbonyl)phthalimide

A. From the acid chloride of Example 2

Into a 125 ml reaction flask equipped with a magnetic stirrer, thermometer, condenser and nitrogen atmosphere were placed 2.1g (0.013 mol) of 2,2,6,6-tetramethyl-4-aminopiperidine and 50 ml of 2% dimethylformamide in methylene chloride. To this was added triethylamine (2.8g, 0.028 mol). This mixture was stirred and cooled during the addition of 5.0g (0.013 mol) of acid chloride of Example 2 accompanied by an exotherm to 40° C. The reaction was allowed to stir at room temperature for 1 hour. The mixture was transferred to a separatory funnel with 100 ml of methylene chloride, 150 ml of water and 50 ml of 5% sodium hydroxide. The phases were separated and the aqueous phase extracted with an additional 50 ml of methylene chloride. The organic solutions were combined and washed with three 100 ml portions of water. The organic solution was dried with anhydrous magnesium sulfate and the solvent stripped using aspirator and high vacuum systems. The product was 1.1g of a white solids. This solid was recrystallized from toluene to give 0.8g of white crystals, melting 260-262° C. The infrared spectrum (chloroform) showed the carbonyl absorption at 1710 $cm^{-1}$ (imide), and 1665 $cm^{-1}$ (amide). The NMR spectrum showed the anticipated aromatic ring (7.7-7.9 ppm, doublet, 1H; 8.0-8.3, multiplet, 2H), the amide N-H (5.9-6.1 ppm, doublet, 1H) and the HALS groups [4.2-4.9 ppm, multiplet, 2H; 0.8-2.3 ppm, several multiplets including two singlets (1.2 and 1.3 ppm)].

B. From trimethyl 1,2,4-benzenetricarboxylate

Into a 100 ml flask equipped with reflux condenser and oil bath were combined 2,2,6,6-tetramethyl-4-aminopiperidine (2,7 g, 0.017 mole), trimethyl 1,2,4-benzenetricarboxylate (2.0 g, 0.008 mole), potassium t-butoxide (0.04g) and 50 ml of mesitylene. This mixture was refluxed for 28 hours, cooled and transferred to a separatory funnel with 100 ml of methylene chloride. The organic solution was washed with three 50 ml portions of water and dried with anhydrous magnesium sulfate. The solvent was stripped using aspirator and high vacuum systems. The residue was mixed with 50 ml of pentane and set aside for crystallization of the product. The crystallized product was isolated by filtration yielding 0.6 g of white crystals. This produce was recrystallized from toluene producing 0.1 g of white crystals with melting point and infrared spectrum in agreement with that obtained for product from procedure A above.

## EXAMPLE 10

Preparation of styrene/allyl alcohol/N-(2,2,6,6-tetramethyl-4-piperidinyl)-4-allyloxycarbonylphthalimide copolymer

Into a 125 ml reaction flask equipped with magnetic stirrer, thermometer, condenser and nitrogen atmosphere were placed 5.0g (0.013 mol) of acid chloride of Example 2 and 50 ml of 2% dimethylformamide in methylene chloride. This mixture was stirred during the addition of 2.9g (about 0.013 hydroxy equivalents) of RJ-101 (a styrene/allyl alcohol copolymer from Monsanto having 7.7±.3 wt % hydroxyl content). To this was added 2.8g (0.028 mol)triethylamine. The reaction was refluxed for 1.5 hours. The mixture was transferred to a separatory funnel with 150 ml of methylene chloride and 100 ml of 5% sodium hydroxide. The phases were separated and the organic phase was further extracted with three 50 ml portions of 5% sodium hydroxide and 50 ml water. The organic solution was dried with anhydrous magnesium sulfate and the solvent stripped using aspirator and high vacuum systems. The product was 4.3g of light yellow crystals. The solid was redissolved in a minimum amount of methylene chlroide and precipitated by dropwise addition to 800 ml of hexane. The solid was isolated by filtration yielding 3.9g. The infrared spectrum (KBr pellet) showed the carbonyl absorption at 1715 $cm^{-1}$ (imide and ester). The glass transition temperature (Tg) was determined to be 87.6° C. Molecular weight data obtained from gel permeation chromatography indicated an Mn of 1400 and an Mw of 2100 (based on polystyrene standard). The NMR spectrum demonstrated the presence of the HALS moiety in the high molecular weight material (two singlets at 1.2 and 1.3 ppm) and the presence of aromatic hydrogens from the pendant phenyl groups.

EXAMPLE 11

Preparation of 4,4′-[2,5-dioxa-1,6-dioxohexane-1,6-diyl]bis[N-(2,2,6,6-tetramethyl-4-piperidinyl)phthalimide]

Into a 125 ml reaction flask equipped with a magnetic stirrer, thermometer, condenser and nitrogen atmosphere were placed 6.5g (0.017 mol) of acid chloride of Example 2 and 50 ml of 2% dimethylformamide in methylene chloride. This mixture was stirred during the addition of 0.43g (0.007 mol) of ethylene glycol. To this was added 7.3g (0.072 mol) triethylamine, accompanied by an exotherm to 40°C. The reaction was refluxed for 4 hours. Acid chloride (1.0g, 0.003 mol) was added and the reflux continued for and additional 2 hours. The mixture was cooled and transferred to a separatory funnel with 50 ml methylene chloride and extracted with one 100 ml portion of 5% sodium hydroxide, two 50 ml portions of 5% sodium hydroxide and three 50 ml portions of water. The organic solution was dried with anhydrous magnesium sulfate and the solvent stripped using aspirator and high vacuum systems. The solid residue was recrystallized from 95% ethanol. The recrystallized solid was dissolved in methylene chloride and dried again with anhydrous magnesium sulfate. The solvent was stripped using aspirator and high vacuum systems yielding 2.5g of yellow solid melting at 70°C. The infrared spectrum (KBr pellet) showed the carbonyl absorption at 1715 $cm^{-1}$ (imide and ester). The NMR spectrum showed the anticipated aromatic ring (7.7-7.9 ppm, doublet, 1H; 8.3-8.5, multiplet, 2H), the HALS N-C-H and the methylenes merged (4.2-5.0 ppm, multiplet with prominent singlet at 4.7 ppm) and the HALS groups [1.1-2.3 ppm, several multiplets including two singlets (1.2 and 1.3 ppm)].

This same product can be prepared from ethylene bis trimellitate (AC-32, a product of Anhydride and Chemical Incorporated), using the procedure described in Example 30. The commercial bis anhydride is a mixture and the product obtained is not as pure as that prepared above.

EXAMPLE 12

Preparation of 4,4′-[1,3-phenylenedi(oxycarbonyl)]-bis[N-2,2,6,6-tetramethyl-4-piperidinyl]phthalimide

Into a 125 ml reaction flask equipped with a magnetic stirrer, thermometer, condenser and nitrogen atmosphere were placed 5.0g (0.013 mol) of acid chloride of Example 2 and 50 ml of 2% dimethylformamide in methylene chloride. This mixture was stirred during the addition of 1.43g (0.013 mol) of resorcinol. To this was added 2.8g (0.028 mol) triethylamine, accompanied by a small exotherm, but the temperature was kept between 20 and 25°C by using an ice bath. The reaction was stirred at room temperature for 2 hours. The mixture was transferred to a separatory funnel with 50 ml water, the phases were allowed to separate and the aqueous solution was washed with 50 ml of fresh methylene chloride. The combined organic solutions were washed with two 50 ml portions of 5% sodium hydroxide. The organic material was then dried with anhydrous magnesium sulfate and the solvent stripped using aspirator and high vacuum systems. The product was 1.8g of a yellow solid melting at 130-135°C. The infrared spectrum (KBr pellet) showed two carbonyl absorptions at 1710 and 1745 $cm^{-1}$ (imide and ester). The NMR spectrum showed the anticipated aromatic rings (7.8-8.0 ppm, doublet, 1H; 8.4-8.6, multiplet, 2H; 7.0-7.6 ppm, multiplet with prominent peak at 7.2 ppm), the HALS group [4.4-4.9 ppm, multiplet, 1H; 1.1-2.3 ppm, several multiplets including two singlets (1.2 and 1.3 ppm)]. The UV spectrum showed an absorbance maximum (THF) at 300 nm.

EXAMPLE 13

Preparation of N-2,2,6,6-tetramethyl-4-piperidinyl)-4-[octylphenoxypoly(ethoxy)carbonyl]phthalimide

Into a 125 ml reaction flask equipped with a magnetic stirrer, thermometer, condenser and nitrogen atmosphere were placed 5.0g (0.013 mol) of acid chloride of Example 2, 50 ml of 2% dimethylformamide in methylene chloride and Triton X-100 (octylphenoxypoly(ethoxy)ethanol, a product of Rohm and Haas Co., 4.6g). To this was added 1.5g (0.015 mol) triethylamine, accompanied by a small exotherm. The reaction cleared then a solid formed as the mixture was heated to reflux. The reaction was refluxed for 4 hours then cooled to room temperature and transferred to a separatory funnel with 50 ml methylene chloride and 50 ml of 5% sodium hydroxide. The phased were allowed to separate and the aqueous solution was washed with three 100 ml portions of fresh methylene chloride. The organic material was then dried with anhydrous magnesium sulfate and the solvent stripped using aspirator and high vacuum systems. The product was 6.0g of a viscous liquid. The infrared spectrum (between salt plates) showed two carbonyl absorptions at 1710 and 1685 $cm^{-1}$ (imide and ester). The NMR spectrum showed the anticipated aromatic rings (7.8-8.0 ppm, doublet; 8.3-8.6, multiplet; 6.7-7.4 ppm, multiplet). The ethylene groups were a prominent singlet at 4.7 (with several smaller peaks at the base) and the HALS and octyl groups were present [0.6-2.3 ppm, several multiplets including a singlet at 0.8 ppm]. The integration of this spectrum indicated that the sample contained considerable unreacted starting alcohol, a crude assay based on this integration indicated the title compound present in about 50%.

## EXAMPLE 14

Preparation of N-(2,2,6,6-tetramethyl-4-piperidinyl)-4-(allyloxycarbonyl)phthalimide

Into a 125 ml reaction flask equipped with a magnetic stirrer, thermometer, condenser and nitrogen atmosphere were placed 5.0g (0.013 mol) of acid chloride of Example 2, 50 ml of 2% dimethylformamide in methylene chloride and allyl alcohol (0.7g, 0.012 mol). To this was added triethylamine (2.8g, 0.028 mol) accompanied by an exotherm from room temperature up to 35° C. The reaction was cooled to room temperature and stirred for 1.5 hours then transferred to a separatory funnel with 50 ml methylene chloride and 50 ml of 5% sodium hydroxide. The phases were allowed to separate and the aqueous solution was washed with an additional 50 ml of fresh methylene chloride. The combined organic solutions were then dried with anhydrous magnesium sulfate and the solvent stripped using aspirator and high vacuum systems. The product was 3.8g of a yellow solid melting at 90-93° C. The solid was recrystallized in methyl t-butyl ether producing almost white crystals melting at 96-98° C. The infrared spectrum (KBr pellet) showed the carbonyl absorption at 1715 $cm^{-1}$ (imide and ester). The NMR spectrum showed the anticipated aromatic ring (7.7-7.9 ppm, doublet, 1H; 8.2-8.5, multiplet, 2H), a typical allyl group pattern merging with the N-C-H of the HALS group (4.8-6.3 ppm for allyl and 4.4-4.9 ppm for HALS, several multiplets, 6H) and the rest of the HALS group [0.8-2.3 ppm, several multiplets including two singlets (1.2 and 1.3 ppm)].

## EXAMPLE 15

Reaction of Jeffamine® D-230 with the acid chloride of Example 2

Into a 125 ml reaction flask equipped with a magnetic stirrer, thermometer, condenser and nitrogen atmosphere were placed 5.0g (0.013 mol) of acid chloride of Example 2, 50 ml of 2% dimethylformamide in methylene chloride and Jeffamine D-230 (an amine terminated polypropylene glycol, product of Texaco Chemical Co., 1.5g). The amine addition caused a small exotherm. To this was added triethylamine (2.8g, 0.028 mol) accompanied by an exotherm from room temperature up to 35° C. The reaction was stirred at

ambient temperature for 2.5 hours. The mixture was transferred to a separatory funnel with 100 ml methylene chloride and 100 ml of 5% sodium hydroxide. The phases were allowed to separate and the organic was washed with two 50 ml portions of 5% sodium hydroxide and three 50 ml portions of water. The organic solution was then dried with anhydrous magnesium sulfate and the solvent stripped using aspirator and high vacuum systems. The product was 4.8g of white crystals. The infrared spectrum (KBr pellet) showed two carbonyl absorptions at 1710 and 1650 $cm^{-1}$ (imide and amide). The Tg was determined to be 61.6 °C. The NMR spectrum showed the anticipated aromatic ring (7.7-7.9 ppm, doublet; 8.0-8.3, multiplet); and the presence of the HALS group indicated by two singlets at 1.2 and 1.3 ppm.

## EXAMPLE 16

### Reaction of the Acid Chloride of Example 2 with Poly(butadiene) diol

Into a 125 ml reaction flask equipped with a magnetic stirrer, thermometer, condenser and nitrogen atmosphere were placed 1.9g (0.005 mol) of acid chloride of Example 2, 50 ml of 2% dimethylformamide in methylene chloride and polybutadiene, diol (nominal M. W. 2800, a product of Scientific Polymer Products, Inc.) (5.0g). To this was added triethylamine (1.0g, 0.01 mol) accompanied by a small exotherm. The reaction was stirred at room temperature for 2 hours. The mixture was transferred to a separatory funnel with 100 ml of methylene chloride and washed with three 50 ml portions of 5% sodium hydroxide (making the best possible separations of the emulsified mixture). The aqueous washes were combined and further diluted with 100 ml of water and extracted with 250 ml of methylene chloride. The organic solutions were combined then dried with anhydrous magnesium sulfate and the solvent stripped using aspirator and high vacuum systems. The product was 5.4g of viscous dark yellow liquid. The infrared spectrum (between salt plates) showed several carbonyl absorptions with a broad major absorption at 1710 $cm^{-1}$. The NMR spectrum demonstrated the presence of the aromatic rings and intense methylene absorbances from the polybutadiene. The presence of the HALS group was indicated by two singlets at 1.2 ppm and 1.3 ppm.

## EXAMPLE 17

### Preparation of N-(2,2,6,6-tetramethyl-4-piperidinyl)-4-[(4-morpholinyl)carbonyl]phthalimide

Into a 125 ml reaction flask equipped with a magnetic stirrer, thermometer, condenser and nitrogen atmosphere were placed 5.0g (0.013 mol) of acid chloride of Example 2 and 50 ml of 2% dimethylformamide in methylene chloride. To this was added morpholine (4.0g, 0.046 mol) accompanied by a strong exotherm, and an ice water bath was used to maintain the reaction temperature below 30 °C. After complete addition of the morpholine, the reaction was stirred at room temperature for 1 hour then transferred to a separatory funnel with 100 ml methylene chloride and washed with three 50 ml portions of 5% sodium hydroxide and three 50 ml portions of water. The organic solution was dried with anhydrous magnesium sulfate and the solvent stripped using aspirator and high vacuum systems. The product was 4.4g of white solids. This material was recrystallized from hexane providing 2.3g of white crystals melting 179-184 °C. The infrared spectrum (KBr pellet) showed two carbonyl absorptions at 1710 and 1640 $cm^{-1}$ (imide and amide). The NMR spectrum showed the anticipated aromatic ring (7.5-8.0 ppm, multiplet, 3H), the morpholine ring (3.3-4.0 ppm, broad singlet, 8H) and the HALS group [4.4-4.9 ppm, multiplet, 1H; 0.9-2.3 ppm, several multiplets including two singlets (1.2 and 1.3 ppm)].

## EXAMPLE 18

Preparation of N-(2,2,6,6-tetramethyl-4-piperidinyl)-4-{[4-(2,2-di(methoxycarbonyl)ethenyl)phenoxy]-carbonyl}phthalimide

Into a 125 ml reaction flask equipped with a magnetic stirrer, thermometer, condenser and nitrogen atmosphere were placed 1.9g (0.005 mol) of acid chloride of Example 2, 50 ml of 2% dimethylformamide in methylene chloride, dimethyl (p-hydroxybenzylidene)malonate (2.6g, 0.011 mol) and triethylamine (1.0g, 0.01 mol). The reaction was refluxed for 3 hours then cooled and transferred to a separatory funnel with 100 ml methylene chloride and washed with three 50 ml portions of 5% sodium hydroxide and three 50 ml portions of water. The organic solutions were combined then dried with anhydrous magnesium sulfate and the solvent stripped using aspirator and high vacuum systems. The product was 5.7g of slightly yellow solid. This material was heated with a solvent mixture of 20% 2-propanol and 80% hexane; then it was cooled and the insoluble material was isolated by filtration (2.8g of yellow solids were collected, melting about 135°C). This was slurried with hot tetrahydrofuran and filtered hot. The tetrahydrofuran was stripped to give 2.4g of slightly yellow solids melting at 55-58°C. The infrared spectrum (chloroform) showed a broad carbonyl absorption at 1710 $cm^{-1}$ (imide and ester). The UV spectrum showed an absorbance maximum at 292 nm with molar absorptivity 20000.

## EXAMPLE 19

Preparation of N-(2,2,6,6-tetramethyl-4-piperidinyl)-4-{[2-hydroxy-3-(alkyl($C_{12}$-$C_{14}$)oxy)propoxy]-carbonyl}phthalimide

Into a 125 ml reaction flask equipped with a magnetic stirrer, thermometer, condenser and nitrogen atmosphere were placed the HALS-acid of Example 1 (7.0g (0.021 mol), Epoxide 8 (a mixture of 1,2-epoxy-3-(alkyl(of 12-14 carbons)oxy)propane, product of Procter and Gamble) (4.3g), Adogen 464 (methyl-trialkyl (of 8-10 carbons) ammonium chloride) (0.11g) and 80 ml of dimethylformamide. The reaction was refluxed for 4 hours and 20 minutes then cooled. The mixture was transferred to a separatory funnel with 200 ml methylene chloride and washed with three 100 ml portions of 5% sodium hydroxide and two 100 ml portions of water. The organic solution was dried with anhydrous magnesium sulfate and the solvent stripped using aspirator and high vacuum systems. The stripped residue still contained residual dimethylformamide and was therefore dissolved in methyl t-butyl ether and washed with three 100 ml portions of water. The organic solution was dried and stripped as before and the residue recrystallized from hexane then a second time from 25% aqueous ethanol. The recrystallized product was dissolved in methylene chloride and dried and stripped as described previously. The product was next dissolved in 10 ml methylene chloride and precipitated into 600 ml pentane. The product was isolated by filtration as 2.7g of white crystals melting 89-92°C. The infrared spectrum showed a broad carbonyl absorption of 1710 $cm^{-1}$ (imide and ester) and a broad hydroxyl and amine absorption of 3300-3700 $cm^{-1}$.

## EXAMPLE 20

Preparation of N-(2,2,6,6-tetramethyl-4-piperidinyl)-4-(n-butoxycarbonyl)phthalimide

Into a 125 ml reaction flask equipped with a magnetic stirrer, thermometer, condenser and nitrogen atmosphere were placed the HALS-acid of Example 1 (3.3g, 0.01 mol), n-butylbromide (3.13g, 0.022 mol), Adogen 464 (0.19g), sodium carbonate (6.64g, 0.044 mol) and 50 ml of dimethylformamide. The reaction was heated to 80-90°C for 2 hours, allowed to stand at ambient temperature for 15 hours then heated again at 80-90°C for 6.5 additional hours. The mixture was transferred to a separatory funnel with 100 ml of

methyl t-butyl ether and 500 ml of water. The phases were agitated and then allowed to separate. The ether solution was retained and the aqueous phase was extracted with 100 ml fresh ether. The combined ether extracts were dried with anhydrous magnesium sulfate and stripped using aspirator and high vacuum systems. The white solid residue was isolated using a small amount of pentane (the product is quite soluble in this solvent). The product was 3.0g of white crystals melting at 90-92 °C. The infrared spectrum (chloroform) showed a broad carbonyl absorption at 1700 $cm^{-1}$ (imide and ester). The NMR spectrum showed the anticipated aromatic ring (7.7-8.5 ppm, multiplet, 3H), the -N-C-H and $-O-CH_2$ merged (4.2-4.8, triplet and multiplet, 3H) and the rest of the HALS group and butyl group [0.9-2.3 ppm, several multiplets including two singlets (1.2 and 1.3 ppm)].

## EXAMPLE 21

### Preparation of N-(2,2,6,6-tetramethyl-4-piperidinyl)-4-(n-octadecyloxcarbonyl)phthalimide

Into a suitable flask were placed 7.0 g of octadecanol, 5.7g of triethylamine, 0.1g of 4-(dimethylamino)-pyridine and about 100 ml of dry methylene chloride. The mixture was stirred during the addition of 10.0g of the acid chloride of Example 2 over a 10 minute period. The reaction was continued at room temperature for 2 hours. The resulting suspension was suction filtered to remove precipitated salt which was washed with fresh methylene chloride. The combined methylene chloride filtrates were placed in a separatory funnel and washed with dilute aqueous hydrochloric acid, water and finally with 5% sodium hydroxide. The solution was dried using anhydrous magnesium sulfate and the solvent stripped to yield 14.6g (97% of theoretical) of a yellow wax. The infrared spectrum of this material showed two carbonyl absorptions (imide and ester) at 1715 and 1725 $cm^{-1}$. The material was examined using liquid chromatography which indicated approximately 4.5% residual octadecanol contaiminated the product.

## EXAMPLE 22

### Reaction of Jeffamine™ M-300 with the Acid Chloride of Example 2

Into a suitable flask were placed 4.5g of Jeffamine M-300 [an ether and amine terminated polypropylene oxide, a product of Texaco Chemical Company], 3.4g of triethylamine, 0.1g of 4-(dimethylamino) pyridine and about 100 ml of dry methylene chloride. The mixture was stirred during the addition of 5.0g of the acid chloride of Example 2 over a 10 minute period. The reaction was allowed to continue at room temperature for 2 hours. The resulting suspension was suction filtered to remove precipitated salt which was washed with fresh methylene chloride. The combined methylene chloride filtrates were placed in a separatory funnel and washed with dilute aqueous hydrochloric acid, water and finally with 5% sodium hydroxide. The solution was dried using anhydrous magnesium sulfate and the solvent stripped to yield 8.3g (99% of theoretical) of a yellow oil. The infrared spectrum of this material showed two carbonyl absorptions (imide and amide) at 1711 $cm^{-1}$ and 1645 $cm^{-1}$ (broad). The material was examined using liquid chromatography which showed that the high molecular weight material has acquired a UV absorption, an indication that the reaction has proceeded as expected.

## EXAMPLE 23

Reaction of the Acid Chloride of Example 2 with Duracarb® 120

Into a suitable flask were placed 6.1g of Duracarb 120 [a hydroxy-terminated aliphatic polycarbonate product of PPG Industries, having a 4.2 wt% hydroxyl groups], 3.4g of triethylamine, 0.1g of 4-(dimethylamino)pyridine and about 100 ml of dry methylene chloride. The mixture was stirred during the addition of 5.0g of the acid chloride of Example 2 over a 10 minute period. The reaction was allowed to continue at room temperature for 2 hours. The resulting suspension was suction filtered to remove precipitated salt which was washed with fresh methylene chloride. The combined methylene chloride filtrates were placed in a separatory funnel and washed with dilute aqueous hydrochloric acid, water and finally with 5% sodium hydroxide. The solution was dried using anhydrous magnesium sulfate and the solvent stripped to yield 9.5g (95% of theoretical) of a viscous yellow oil. The infrared spectrum of this material showed two carbonyl absorptions (imide and ester) at 1715 and 1740 $cm^{-1}$. The material was examined using liquid chromatography which showed that the high molecular weight material had acquired an enhanced UV absorption, an indication that the reaction had proceeded as expected.

EXAMPLE 24

Reaction of the Acid Chloride of Example 2 with Tone™ 220

Into a suitable flask were placed 7.5g of Tone 220 [a hydroxy-terminated aliphatic polycaprolactone product of Union Carbide Comapny, having an assay of 3.4 wt% hydroxyl groups], 3.2g of triethylamine, 0.1g of 4-(dimethylamino)pyridine and about 100 ml of dry methylene chloride. The mixture was stirred during the addition of 5.0g of the acid chloride of Example 2 over a 10 minute period. The reaction was allowed to continue at room temperature for 2 hours. The resulting suspension was suction filtered to remove precipitated salt which was washed with fresh methylene chloride. The combined methylene chloride filtrates were placed in a separatory funnel and washed twice with dilute aqueous hydrochloric acid. During the second wash an emulsion formed that was broken with the addition of sodium chloride. The aqueous acidic washes were back-extracted with fresh methylene chloride. The combined organic solutions were then washed with potassium bicarbonate solution. The solution was dried using anhydrous magnesium sulfate and the solvent stripped to yield 7.1g (62% of theoretical) of a yellow oil. The infrared spectrum of this material showed a broad carbonyl absorption (imide and ester) at 1700-1750 $cm^{-1}$. The material was examined using liquid chromatography which showed that the high molecular weight material had acquired an enhanced UV absorption, an indication that the reaction had proceeded as expected.

EXAMPLE 25

Reaction of the Acid Chloride of Example 2 with Poly(ethylene glycol), methyl ether

Into a suitable flask were placed 5.0g of poly(ethylene glycol), methyl ether [approximate molecular weight of 350], 3.6g of triethylamine, 5.5g of the acid chloride of Example 2 and 75 ml of acetone. The reaction was stirred and warmed to a reflux which was continued for a short period. The reaction was allowed to stand at room temperature over night. The resulting suspension was suction filtered to remove precipitated salt and stripped. The residual oil was dissolved in methylene chloride and washed twice with water. The solution was dried using anhydrous magnesium sulfate and the solvent stripped to yield 7.7g

(82% of theoretical) of a viscous yellow oil. The infrared spectrum of this material showed a carbonyl absorption (imide and ester) at 1715 $cm^{-1}$ and a broad C-O absorption at 1100-1110 $cm^{-1}$. The material was examined using liquid chromatography which showed that the high molecular weight material had acquired an enhanced UV absorption, an indication that the reaction had proceeded as expected.

## EXAMPLE 26

Accelerated Weathering of ABS containing N-(2,2,6,6-tetramethyl-4-piperidinyl)-4-(2,2,6,6-tetramethyl-4-piperidinyloxycarbonyl)phthalimide .

A. Sample Preparation

ABS polymer (Dow 500 natural, dried for 4 hours at 90°C) was used to prepare test specimens for weathering. The ABS compositions prepared were:

1. 0.42 phr HALS of Example 5 and 0.25 phr Tinuvin P
2. 0.25 phr Tinuvin P
3. 0.50 phr Tinuvin P
4. none (control)

The additives were first mixed with the polymer and then the blend was extruded twice in a Brabender Prep Center Extruder at 230°C. The control was extruded twice even though no stabilizer was added. The resulting stabilized polymer was pelletized and molded into plaques on a Newbury 25 ton injection molder at 400°F.

B. Weathering Test

The plaques prepared above were weathered in an Atlas Ci65 Weather-O-Meter using a 6500 watt xenon arc source (inner and outer borosilicate filters, irradiance 0.38 $w/m^2$ at 340 nm) and a cycle time of 3 hours (2 hours of light at 70°C black panel temperature with 50% relative humidity and a 20 minute front spray during the first hour, and 1 hour dark period at 38°C with 100% relative humidity and continual back spray). The samples were removed from the test instrument and color development was measured on a Gardner colorimeter. Increase in yellowness index ( YID) was monitored, and the values were obtained from an average of three samples. The results are shows in Table I. These results demonstrate that the use of a hindered amine of this invention in conjunction with a known UV absorber enhances the stability of ABS to accelerated weathering. The effect of the hindered amine (0.42 phr) is distinct from that of the UV absorber (0.25 phr) as demonstrated by controls which show that the UV absorber alone (0.25 phr) and at twice the level (0.50 phr) do not provide the enhanced degree of stabilization observed when the hindered amine is present.

TABLE I

| Accelerated Weathering of ABS Containing HALS | | | | | | | |
|---|---|---|---|---|---|---|---|
| Stabilized Polymer Composition | YID after exposure (days) | | | | | | |
| | 2 | 7 | 14 | 21 | 28 | 35 | 48 |
| 1 | 18.9 | 14.2 | 14.3 | 14.4 | 16.1 | 18.0 | 21.9 |
| 2 | 18.7 | 13.6 | 14.1 | 14.8 | 17.2 | 19.6 | 24.9 |
| 3 | 19.2 | 14.6 | 16.3 | 17.6 | 20.7 | 23.6 | 29.7 |
| 4. | 24.0 | 26.8 | 31.8 | 35.1 | 39.0 | 42.8 | 47.9 |

## EXAMPLE 27

### Preparation of N-(2,2,6,6-tetramethyl-4-piperidinyl)-4-[(2-hydroxy-2-phenylethoxy)carbonyl]phthalimide

Into a 125 ml reaction flask equipped with a magnetic stirrer, thermometer, condenser and nitrogen atmosphere were placed the HALS of Example 1 (5.0g, 0.015 mol), styrene oxide (1.6g, 0.013 mol). Adogen 464 (Ashland Chemical Co.) (0.15g) and 60 ml of dimethylformamide. The reaction was refluxed for 5 hours and then cooled. The mixture was transferred to a separatory funnel with 100 ml of methylene chloride and washed with 200 ml of 2.5% sodium hydroxide. Additional methylene chloride, 100 ml, was added and the solution was extracted with two 100 ml portions of 5% sodium hydroxide and then with 100 ml of water. The organic solution was then dried with anhydrous magnesium sulfate and the solvent was stripped using aspirator and high vacuum systems. The solid residue (2.9g) was recrystallized from aqueous ethanol then recrystallized again from methyl t-butyl ether/hexane. After high vacuum removal of residual solvent, the product weighed 0.6g and melted at 144-146 °C. The infrared spectrum (in chloroform) showed a broad carbonyl absorption at 1710 $cm^{-1}$ (imide and ester). The structure of this material was further confirmed using proton NMR spectroscopy.

## EXAMPLE 28

### Preparation of 4,4′-[4,13-dihydroxy-1,16-dioxo-2,6,11,15-tetraoxahexadecane-1,16-diyl] bis [N-(2,2,6,6-tetramethyl-4-piperidinyl)phthalimide]

Into a 125 ml reaction flask equipped with a magnetic stirrer, thermometer, condenser, and nitrogen atmosphere were placed the HALS of Example 1 (4.3g, 0.013 mol), 1,4-butane diol diglycidyl ether (1.3g, 0.006 mol), Adogen 464 (Ashland and Chemical Co.) (0.13g) and 50 ml of dimethylformamide. The reaction was refluxed for 5 hours and then filtered. The filtered mixture was poured into 200 ml of water producing a precipitate. The solution was stirred for 5 minutes; then the solids were collected by filtration, slurried with another 500 ml of water, stirred 30 minutes and then isolated again. The wet solids were dissolved in tetrahydrofuran and dried with anhydrous magnesium sulfate. The solvent was stripped and the resulting light tan crystals (4.0g) were recrystallized from about 75 ml of 95% ethanol. The recrystallized solids were

dissolved in tetrahydrofuran, dried and stripped as before with additional removal of solvent under high vacuum. The result was 2.4g of white crystals melting at 117-122° C. The infrared spectrum (in chloroform) showed a broad carbonyl absorption at 1710 $cm^{-1}$ (imide and ester) and a broad OH and NH absorption at 3100-3600 $cm^{-1}$.

EXAMPLE 29

Preparation of N-(1-acetyl-2,2,6,6-tetramethyl-4-piperidinyl)-4-(ethoxycarbonyl)phthalimide

Into a 125 ml reaction flask equipped with a magnetic stirrer, thermometer, condenser and nitrogen atmosphere were placed the HALS of Example 4 (0.9g (0.0025 mol), 4-dimethylaminopyridine (0.03g), and 25 ml of acetic anhydride. The reaction was refluxed for 2 hours then poured into a breaker containing 200 ml ice water. A brown oil separated immediately and the mixture was stirred until the oil solidified into a tan solid. This solid was isolated by filtration and washed on the filter funnel with several portions of water. The wet solid was dissolved in 50 ml methylene chloride and dried with anhydrous magnesium sulfate. The solvent was stripped to give 0.9g of light tan crystals melting 119-121° C. The infrared spectrum (in chloroform) showed a broad carbonyl absorption at 1710 $cm^{-1}$ (imide and ester) and an amide carbonyl at 1615 $cm^{-1}$.

EXAMPLE 30

Preparation of 4,4′-[4-acetoxy-2,6-dioxa-1,7-dioxoheptane-1,7-diyl) bis[N-(1-acetyl-2,2,6,6-tetramethyl-4-piperidinyl)phthalimide]

1. Reaction of glycerol acetate bis trimellitate with 2,2,6,6-tetramethyl-4-aminopiperidine

Into a 1 liter flask equipped with mechanical stirrer, and nitrogen atmosphere were combined glycerol acetate bis trimellitate (33.8 g, 0.07 mole, this ester is available commercially as AC-32, a product of Anhydrides and Chemicals Incorporated) and dimethylformamide (200 ml). This mixture was warmed to 50° C to dissolve the anhydride and 2,2,6,6-tetramethyl-4-aminopiperidine (25.0 g, 0.16 mole) was added which formed an immediate precipitate. The reaction was stirred for 2 hours while cooling to ambient temperature. Methyl t-butyl ether (400 ml) was added to the mixture and the solid product was isolated by filtration. The filtered solid was slurried three times with 300 ml portions of ether and filtered each time. The isolated solid was placed under high vacuum and heated to about 170° C for 1 hour. The resulting white powder weighted 54.6 g and melted at 203-207° C. By infrared spectroscopy, this material was identified as the bis amic acid (internal salt) with ester carbonyl banding at 1715 $cm^{-1}$, amide and acid salt carbonyl banding at 1540-1620 $cm^{-1}$. Upon heating to 270° C, this amic acid further reacted to form the bis imide. The infrared spectrum having a broad intense carbonyl band at about 1710 $cm^{-1}$ and only small residual banding in the region of 1500-1680 $cm^{-1}$.

2. Preparation of the title compound

Into a 250 ml flask equipped with reflux condenser were placed the amic acid prepared above (10.0 g, 0.013 mole) and 100 ml of acetic anhydride. The mixture was refluxed for 2 hours, during which time the amic acid slowly went into solution. The reaction mixture was poured into a beaker packed with ice and

brought to pH 14 using 45% potassium hydroxide (aqueous). A brown gum separated from solution and was isolated by decanting away the aqueous solution. The gum was dissolved in methylene chloride and transferred to a separatory funnel where it was washed with 10% aqueous sodium hydroxide, 5% aqueous hydrochloric acid and saturated aqueous sodium bicarbonate. The organic phase was dried with anhydrous magnesium sulfate and the solvent was stripped using an aspirator and high vacuum systems yielding 5.6 g of a light tan solid. The infrared spectrum of this material showed an intense carbonyl absorption at 1710 $cm^{-1}$ (ester and imide) and another carbonyl absorption at 1620 $cm^{-1}$ (amide).

## Claims

1. A hindered amine compound of the general formula (I)

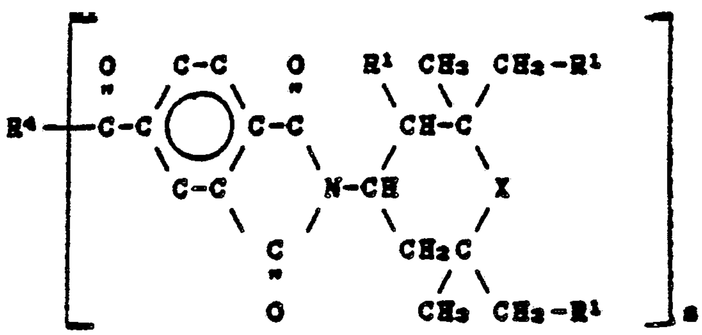

(I)

where $R^1$ is selected from hydrogen, substituted or unsubstituted aliphatic of 1-20 carbons, substituted or unsubstituted aryl of 6-10 carbons, and substituted or unsubstituted araliphatic of 7-22 carbons,
X is selected from

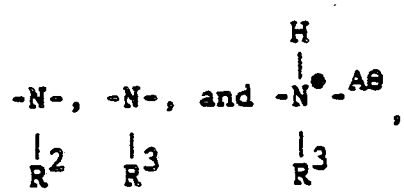

and

$R^2$ is selected from oxyl, hydroxy, and substituted or unsubstituted aliphatic acyl of 1-20 carbons, substituted or unsubstituted alicyclic acyl of 6-14 carbons, substituted or unsubstituted aryl acyl of 7-22 carbons, substituted or unsubstituted araliphatic acyl of 7-22 carbons, $-C(=O)-N(R^1)(R^5)$, $-C(=O)-O-R^5$,

$R^3$ is selected from hydrogen, substituted or unsubstituted aliphatic of 1-20 carbons, substituted or unsubstituted araliphatic radical of 7-22 carbons, alkoxyalkyl of 2-21 carbons, and $-CH_2-C(=O)-O-R^5$,

$R^4$ is selected from hydroxy, $-O^{\ominus}$, halogen or the residue from a substituted or unsubstituted, mono or polyfunctional alcohol, amine, mercaptan or molecular mixture thereof, including hydroxy-and amine-containing polymers which may be in the backbone, pendant, and terminally functionalized,

$R^5$ is selected from hydrogen, substituted or unsubstituted aliphatic of 1-20 carbons, substituted or unsubstituted aryl of 6-10 carbons, substituted or unsubstituted araliphatic of 7-22 carbons, and substituted or unsubstituted alicyclic of 5-12 carbons which may optionally contain heteroatoms as ring members selected from nitrogen, oxygen and sulfur,

s is an integer of 1-100, and

A is selected from chloride, bromide, sulfate, acid sulfate, sulfite, acid sulfite, p-toluenesulfonate, phenylsulfonate, methylsulfonate, phosphate, acid phosphate, carboxylate from any carboxylic acid, and $R^4$ when $R^4$ is $-O^{\ominus}$ wherein substituents for the aliphatic, aromatic, araliphatic, and alicyclic radicals include one or more groups selected from alkyl of 1-4 carbons, alkoxy of 1 to 4 carbons, acyloxy of 1-12 carbons, alkoxycarbonyl of 2-5 carbons, arylcarbonyl of 7-11 carbons, acryloyloxy, methacryloyloxy, aryloxy of 6-10 carbons, aralkyl of 7-10 carbons, aryloxycarbonyl of 7-11 carbons, aryl of 6-10 carbons, amino, hydroxy, carboxy, nitrile, chloro, bromo, epoxy, alkyl mercapto of 1-4 carbons, aryl mercapto of 6-10 carbons, alkylamino of 1-4 carbons, dialkylamino of 2-8 carbons total, arylamino of 6-10 carbons, aryl alkyl amino of

7-10 carbons, and trialkoxysilyl of 3-9 carbons.

2. The hindered amine compound of claim 1 wherein $R^1$ is selected from hydrogen or alkyl of 1 to 4 carbons, aralkyl of 7-10 carbons or phenyl, $R^2$ is selected from an aliphatic acyl of 2-5 carbons, or substituted or unsubstituted aryl acyl of 7-22 carbons, and $R^3$ is selected from hydrogen, substituted or unsubstituted alkyl of 1-4 carbons, allyl, alkoxyalkyl of 2-6 carbons or aralkyl of 7-8 carbons.

3. The compound of Claim 2 wherein $R^1$ is hydrogen, $R^2$ is acetyl, benzoyl, or 3,5-di-t-butyl-4-hydroxybenzoyl and $R^3$ is hydrogen, substituted or unsubstituted alkyl of 1-4 carbons, allyl or benzyl.

4. The compound of claim 3 wherein when s is 1, $R^4$ is selected from chlorine, bromine, 1-morpholinyl, 1-piperidinyl, 1-pyrrolidinyl, and $R^7$ -Y- in which Y is selected from -O-, -N($R^6$)-, -S-, -NH-C( = O)-NH-NH-, -O-C( = O)-NH-NH-, -NH-C( = O)-C( = O)-NH-NH-, and -O-C( = O)-C( = O)-NH-NH-, and

$R^7$ is selected from $R^5$, trialkylsilylalkyl of 5 to 20 carbons, alkoxyalkyl of 3 to 20 carbons, 3-(2H-benzotriazol-2-yl)-2-hydroxybenzyl, triethoxysilylpropyl, and polyalkyl having the formula $CH_3-(CH_2)_c-$ in which c is an integer of 25 to 50, and

$R^6$ is selected from hydrogen, aliphatic of 1-22 carbons, aryl of 6-10 carbons, araliphatic of 7-22 carbons, alicyclic of 5-10 carbons which may optionally contain at least one heteroatom ring member selected from nitrogen, oxygen, and sulfur.

5. The compound of Claim 3 wherein when s is at least one, $R^4$ is selected from 1,4-piperazindiyl, and -Y-$R^8$-Y-wherein Y is as previously defined, $R^8$ is selected from (i) aliphatic diradical of 2-20 carbons, (ii) alicyclic diradical of 5-20 carbons, (iii) aryl diradical of 6-10 carbons, (iv) araliphatic diradical of 7-22 carbons, (v) alkyldiarylene of 13-20 carbons, (vi) cycloalkyldialkyl of 7-12 carbons, (vii) 4,4′-oxybis-(phenylene)-diyl, (viii) 1,4-phenylenebis(dimethylsilyl), (ix) 1,2- and 1,4-phenylenebis(oxyalkyl) of 10-14 carbons, (x) 1,2-, 1,3-, and 1,4-phenylenebis(alkyl) of 8-16 carbons, (xi) oxybis(dimethylsilylpropyl), (xii) 4,4′-biphenyl-diyl, (xiii) 2,2-propane-bis(phenylene)-p,p′-diyl, (xiv) diphenylsulfone-4,4′-diyl, (xv) poly(alkoxy)-dialkyl having the structure

$$-(CH_2)_d-\underset{R^1}{CH}-(CH_2-\underset{R^1}{CH}-O)_e-(CH_2)_d-\underset{R^1}{CH}-$$

in which d is an integer of 1-2 and e is an integer of 0-350, (xvi) polyesters having the structure

$$-R^9-O-\overset{\overset{O}{\|}}{C}-[(O-R^9-O-\overset{\overset{O}{\|}}{C})_f-O-R^{10}-O-\overset{\overset{O}{\|}}{C}]_g-O-R^{10}-$$

in which f and g are integers independently selected from 1-5, and $R^9$ and $R^{10}$ are independently selected from aliphatic or alicyclic diradical of 2-20 carbons, aromatic or araliphatic diradical of 6-22 carbons, alkyldiarylene of 13-20 carbons, and cycloalkyldialkyl of 7-12 carbons, (xvii) copolyesters having the structure

$$-R^9-O-[\overset{\overset{O}{\|}}{C}-R^{10}-\overset{\overset{O}{\|}}{C}-O-R^9-O]_h-\overset{\overset{O}{\|}}{C}-R^{10}-\overset{\overset{O}{\|}}{C}-O-R^9-$$

in which h is an integer from 0-10, (xviii) unsaturated polyolefins having the structure -[polybutadiene]- in which the polybutadiene has molecular weight of from 2000 to 3500, (xix) poly(mercaptoethers) having the structure

$$-[CH_2-CH_2-S-CH_2-\underset{R^1}{CH}-O]_i-CH_2-CH_2-S-CH_2-\underset{R^1}{CH}-$$

in which i is an integer from 2 to 12, (xx) unsaturated copolymers having the structure

$$-[(\text{polybutadiene})_j-(CH_2-\underset{CN}{CH})_k-]_m-$$

in which m is an integer of 5-10, and j and k are component fractions, k is selected from about 0.1 to 0.3 and j is 1-k, (xxi) bicyclic divalent hydrocarbons having the structure

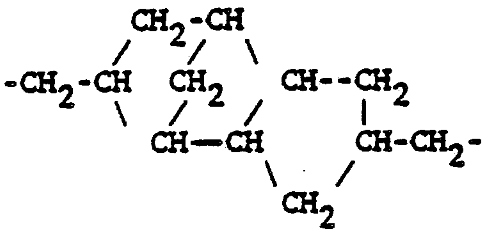
, and

(xxii) poly(organosiloxanes) having the structure

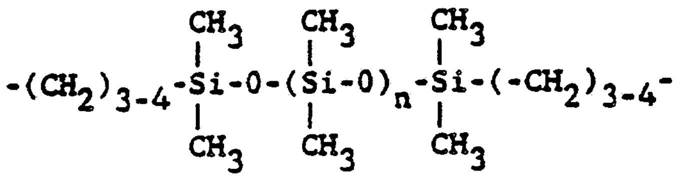
.

in which n is an integer of 5-3000.

6. The compound of Claim 3 wherein when s is at least one, $R^4$ is selected from

(i) polyester having the structure

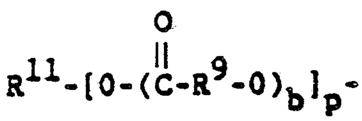

in which b is an integer of 1-10, p is 2 or 3, and when p is 2, $R^{11}$ is aliphatic diradical of 2 to 4 carbons, and when p is 3, $R^{11}$ is aliphatic triradical of 3 to 8 carbons, and

(ii) 4-aza-1,7-dioxaheptane-1,4,7-triyl,

(iii) 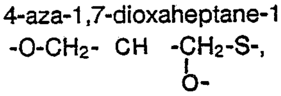

(iv)

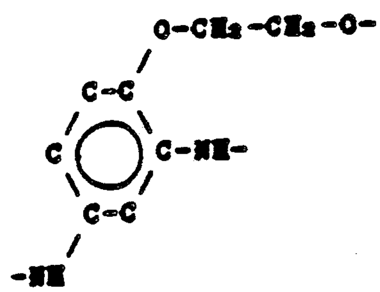

(v)

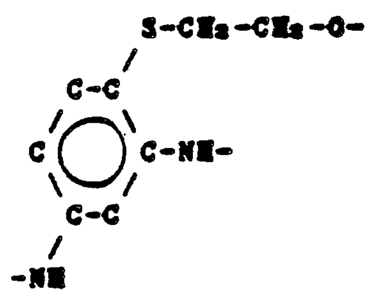

(vi)

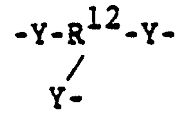

wherein

Y is as previously defined, $R^{12}$ is selected from aliphatic triradical of 3 to 20 carbons, aromatic triradical of 6 to 13 carbons, 1,3,5-triazine-2,4,6-triyl, triisopropylbenzene-alpha, alpha′, alpha″-triyl, nitrillotriethyl,

(vii)

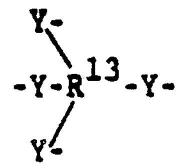

wherein

Y is as previously defined, $R^{13}$ is aliphatic tetraradical of 4 to 20 carbons,

(viii)

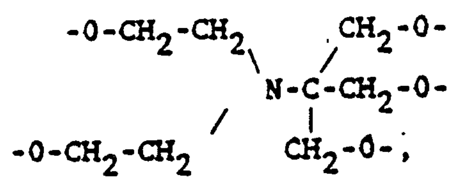

(ix)

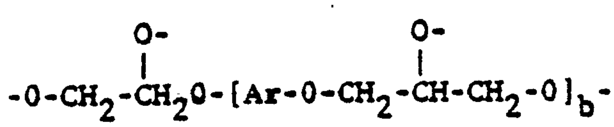 wherein

Ar is 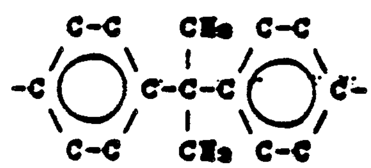

and b is as previously defined, and

(x) $-NH-(CH_2-CH_2-N)_{1-2}-CH_2-CH_2NH-$.

7. The compound of claim 3 wherein when s is at least one, $R^4$ is selected from

(i)

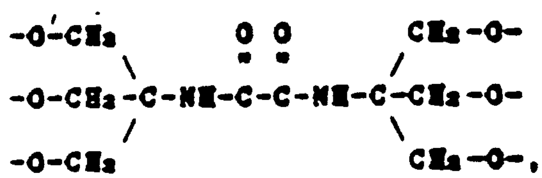

(ii)

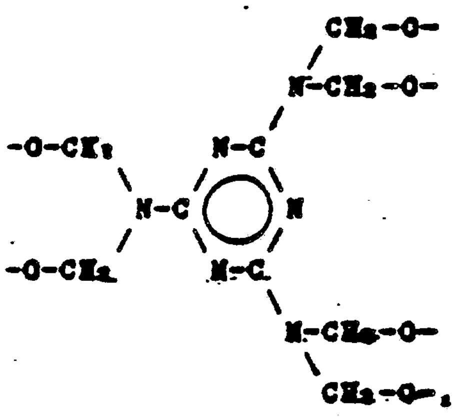

, and

(iii)

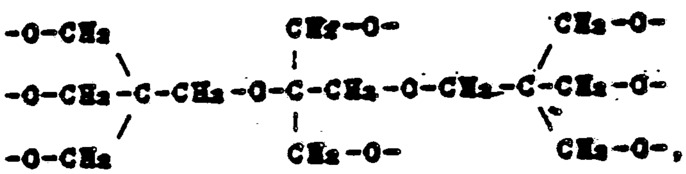

, and

(iv) a polymeric or copolymeric radical containing recurring units

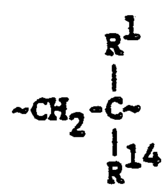

wherein

$R^{14}$ is selected from -O-, -N($R^6$)-, $-CH_2-O-$, $-C(=O)-O-CH_2-CH(OH)-CH_2-O-$, $-C(=O)-O-CH_2-CH_2-O-$, and $-C(=O)-O-CH_2-CH_2-CH_2-O-$, and ~ is the polymer or copolymer backbone in which the units recur.

8. The compound of claim 1 selected from N-(2,2,6,6-tetramethyl-4-piperidinyl)-4-carboxyphthalimide, N-(2,2,6,6-tetramethyl-4-piperidinyl)-4-(chlorocarbonyl)phthalimide, hydrochloride salt, N-(2,2,6,6-tetramethyl-4-piperidinyl)-4-(aminocarbonyl)phthalimide, N-(2,2,6,6-tetramethyl-4-piperidinyl)-4-(ethoxycarbonyl)-phthalimide, N-(2,2,6,6-tetramethyl-4-piperidinyl)-4-(2,2,6,6-tetramethyl-4-piperidinyloxycarbonyl)phthalimide, N-(2,2,6,6-tetramethyl-4-piperidinyl)-4-(2-ethylhexyloxycarbonyl)phthalimide, N-(2,2,6,6-tetramethyl-4-piperidinyl)-4-(dodecylaminocarbonyl)phthalimide, N-(2,2,6,6-tetramethyl-4-piperidinyl)-4-(dibutylaminocarbonyl)phthalimide, N-(2,2,6,6-tetramethyl-4-piperidinyl)-4-(2,2,6,6-tetramethyl-4-piperidinylaminocarbonyl)phthalimide, styrene/N-(2,2,6,6-tetramethyl-4-piperidinyl)-4-(allyl-oxycarbonyl)-phthalimide copolymer 4,4′-[2,5-dioxa-1,6-dioxohexane-1,6-diyl]bis[N-(2,2,6,6-tetramethyl-4-piperidinyl)-phthalimide], 4,4′-[1,3-phenylenedi(oxycarbonyl)]bis[N-(2,2,6,6-tetramethyl-4-piperidinyl)phthalimide], N-(2,2,6,6-tetramethyl-4-piperidinyl)-4-(octylphenoxypoly(ethoxy)carbonyl)phthalimide, N-(2,2,6,6-tetramethyl-4-piperidinyl)-4-(allyloxycarbonyl)phthalimide, N-(2,2,6,6-tetramethyl-4-piperidinyl)phthalimide-4-carbonyl endcapped amine terminated polypropylene glycol, N-(2,2,6,6-tetramethyl-4-piperidinyl)phthalimide-4-carbonyl endcapped polybutadiene diol, N-(2,2,6,6-tetramethyl-4-piperidinyl)-4-[(4-mor pholinyl)carbonyl]phthalimide, N-(2,2,6,6-tetramethyl-4-piperidinyl)-4-{[4-(2,2,-di(methoxycarbonyl)ethenyl)phenoxy]carbonyl}-phthalimide, N-(2,2,6,6-tetramethyl-4-piperidinyl)-4-{[2-hydroxy-3-(alkyl($C_{12}$-$C_{14}$)oxy)propoxy]carbonyl}phthalimide, N-(2,2,6,6-tetramethyl-4-piperidinyl)-4-(butoxycarbonyl)phthalimide, N-(2,2,6,6-tetramethyl-4-piperidinyl)-4-(octadecyl-oxycarbonyl)phthalimide, N-(2,2,6,6-tetramethyl-4-piperidinyl)phthalimide-4-carbonyl endcapped amine terminated polypropylene oxide, N-(2,2,6,6-tetramethyl-4-piperidinyl)phthalimide-4-carbonyl endcapped aliphatic polycaprolactone diol, or N-(2,2,6,6-tetramethyl-4-piperidinyl)-phthalimide-4-carbonyl endcapped poly(ethylene glycol) monomethyl ether, N-(2,2,6,6-tetramethyl-4-piperidinyl)phthalimide-4-carbonyl endcapped hydroxyterminated aliphatic polycarbonate, N-(2,2,6,6-tetramethyl-4-piperidinyl)-4-[(2-hydroxy-2-phenylethoxy)carbonyl]phthalimide, 4,4′-[4,13-di-hydroxy-1,16-dioxo-2,6,11,15-tetraoxa-hexadecane-1,16-diyl] bis[N-(2,2,6,6-tetramethyl-4-piperidinyl)phthalimidel, N-(1-acetyl-2,2,6,6-tetramethyl-4-piperidinyl)-4-(ethoxycarbonyl)phthalimide, 4,4-(4-acetoxy-2,6-dioxa-1,7-dioxoheptane-1,7-diyl) bis[N-(1-acetyl-2,2,6,6-tetramethyl-4-piperidinyl)phthalimide], 4,4′-[2,5-dioxa-1,6-dioxohexane-1,6-diyl] bis[N-(2,2,6,6-tetramethyl-4-piperidinyl)phthalimide], and 4,4′-(4-acetoxy-2,6-dioxa-1,7-dioxoheptane-1,7-diyl) bis[N-(2,2,6,6-tetramethyl-4-piperidinyl)phthalimide].

9. Use of compounds of one of claims 1 to 8 for stabilizing polymers and copolymers.

10. Composition on the basis of at least one polymer or oopolymer, characterized by a content of at least one compound of claims 1 to 8.

Claims for the following contracting State: ES

1. A process for preparing a hindered amine compound of the general formula (I)

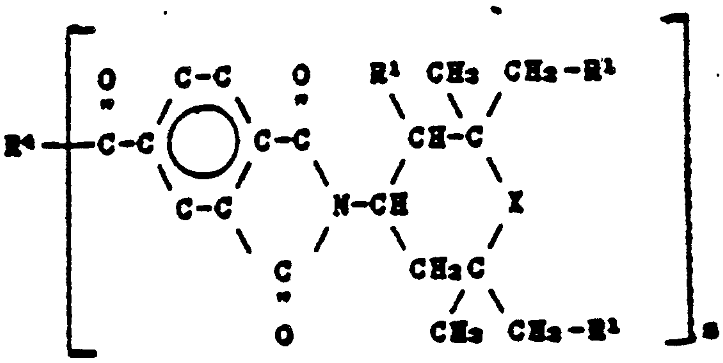

(I)

where $R^1$ is selected from hydrogen, substituted or unsubstituted aliphatic of 1-20 carbons, substituted or unsubstituted aryl of 6-10 carbons, and substituted or unsubstituted araliphatic of 7-22 carbons,
X is selected from

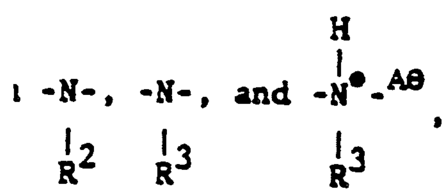

and

$R^2$ is selected from oxyl, hydroxy, and substituted or unsubstituted aliphatic acyl of 1-20 carbons, substituted or unsubstituted alicyclic acyl of 6-14 carbons, substituted or unsubstituted aryl acyl of 7-22 carbons, substituted or unsubstituted araliphatic acyl of 7-22 carbons, $-C(=O)-N(R^1)(R^5)$, $-C(=O)-O-R^5$,

$R^3$ is selected from hydrogen, substituted or unsubstituted aliphatic of 1-20 carbons, substituted or unsubstituted araliphatic radical of 7-22 carbons, alkoxyalkyl of 2-21 carbons, and $-CH_2-C(=O)-O-R^5$,

$R^4$ is selected from hydroxy, $-O^{\ominus}$, halogen or the residue from a substituted or unsubstituted, mono or polyfunctional alcohol, amine, mercaptan or molecular mixture thereof, including hydroxy- and amine-containing polymers which may be in the backbone, pendant, and terminally functionalized,

$R^5$ is selected from hydrogen, substituted or unsubstituted aliphatic of 1-20 carbons, substituted or unsubstituted aryl of 6-10 carbons, substituted or unsubstituted araliphatic of 7-22 carbons, and substituted or unsubstituted alicyclic of 5-12 carbons which may optionally contain heteroatoms as ring members selected from nitrogen, oxygen and sulfur,

s is an integer of 1-100, and

A is selected from chloride, bromide, sulfate, acid sulfate, sulfite, acid sulfite, p-toluenesulfonate, phenylsulfonate, methylsulfonate, phosphate, acid phosphate, carboxylate from any carboxylic acid, and $R^4$ when $R^4$ is $-O^{\ominus}$ wherein substituents for the aliphatic, aromatic, araliphatic, and alicyclic radicals include one or more groups selected from alkyl of 1-4 carbons, alkoxy of 1 to 4 carbons, acyloxy of 1-12 carbons,

alkoxycarbonyl of 2-5 carbons, arylcarbonyl of 7-11 carbons, acryloyloxy, methacryloyloxy, aryloxy of 6-10 carbons, aralkyl of 7-10 carbons, aryloxycarbonyl of 7-11 carbons, aryl of 6-10 carbons, amino, hydroxy, carboxy, nitrile, chloro, bromo, epoxy, alkyl mercapto of 1-4 carbons, aryl mercapto of 6-10 carbons, alkylamino of 1-4 carbons, dialkylamino of 2-8 carbons total, arylamino of 6-10 carbons, aryl alkyl amino of 7-10 carbons, and trialkoxysilyl of 3-9 carbons,

by reacting trimellitic acid derivatives of the formulas

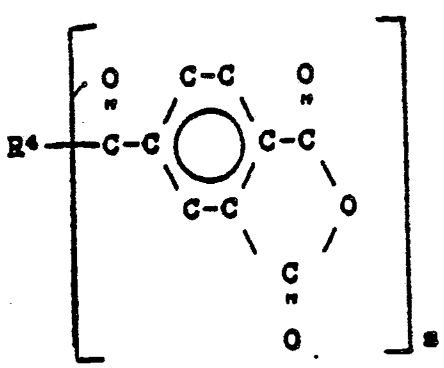

or

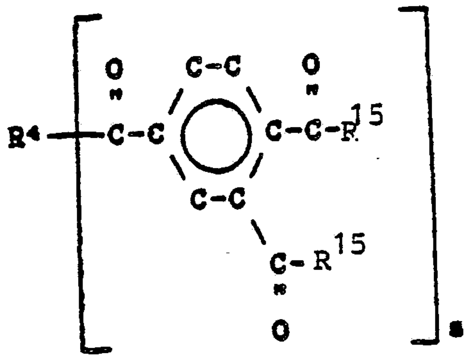

,

wherein $R^{15}$ is selected from $-OR^5$, $N(R^5)_2$ and halogen and $R^4$, $R^5$ and s are as previously defined, with an amino-substituted hindered amine of the formula

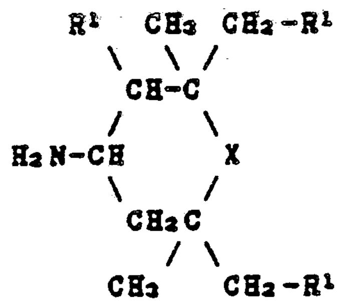

wherein $R^1$ and X are as previously defined, and, if desired, converting the resulting compound of the general formula (I) into another compound of the general formula (I) by reacting radicals $R^4$ and/or X in a manner known per se.

2. The process of claim 1 for preparing a compound of the general formula (I), wherein $R^1$ is selected from hydrogen or alkyl of 1 to 4 carbons, aralkyl of 7-10 carbons or phenyl, $R^2$ is selected from an aliphatic acyl of 2-5 carbons, or substituted or unsubstituted aryl acyl of 7-22 carbons, and $R^3$ is selected from hydrogen, substituted or unsubstituted alkyl of 1-4 carbons, allyl, alkoxyalkyl of 2-6 carbons or aralkyl of 7-8 carbons.

3. The process of claim 1 for preparing a compound as in claim 2, wherein $R^1$ is hydrogen, $R^2$ is acetyl, benzoyl, or 3,5-di-t-butyl-4-hydroxybenzoyl and $R^3$ is hydrogen, substituted or unsubstituted alkyl of 1-4 carbons, allyl or benzyl.

4. The process of claim 1 for preparing a compound as in claim 3, wherein when s is 1, $R^4$ is selected from chlorine, bromine, 1-morpholinyl, 1-piperidinyl, 1-pyrrolidinyl, and $R^7$ -Y- in which Y is selected from -O-, $-N(R^6)-$, -S-, -NH-C(=O)-NH-NH-, -O-C(=O)-NH-NH-, -NH-C(=O)-C(=O)-NH-NH-, and -O-C(=O)-C(=O)-NH-NH-, and

$R^7$ is selected from $R^5$, trialkylsilylalkyl of 5 to 20 carbons, alkoxyalkyl of 3 to 20 carbons, 3-(2H-benzotriazol-2-yl)-2-hydroxybenzyl, triethoxysilylpropyl, and polyalkyl having the formula $CH_3-(CH_2)_c-$ in which c is an integer of 25 to 50, and

$R^6$ is selected from hydrogen, aliphatic of 1-22 carbons, aryl of 6-10 carbons, araliphatic of 7-22 carbons, alicyclic of 5-10 carbons which may optionally contain at least one heteroatom ring member selected from nitrogen, oxygen, and sulfur.

5. The process of claim 1 for preparing a compound as in claim 3, wherein when s is at least one, $R^4$ is selected from 1,4-piperazindiyl, and $-Y-R^8-Y-$wherein Y is as previously defined, $R^8$ is selected from (i) aliphatic diradical of 2-20 carbons, (ii) alicyclic diradical of 5-20 carbons, (iii) aryl diradical of 6-10 carbons,

(iv) araliphatic diradical of 7-22 carbons, (v) alkyldiarylene of 13-20 carbons, (vi) cycloalkyldialkyl of 7-12 carbons, (vii) 4,4′-oxybis(phenylene)-diyl, (viii) 1,4-phenylenebis(dimethylsilyl), (ix) 1,2- and 1,4-phenylenebis(oxyalkyl) of 10-14 carbons, (x) 1,2-, 1,3-, and 1,4-phenylenebis(alkyl) of 8-16 carbons, (xi) oxybis(dimethylsilylpropyl), (xii) 4,4′-biphenyl-diyl, (xiii) 2,2-propane-bis(phenylene)-p,p′-diyl, (xiv) diphenylsulfone-4,4′-diyl, (xv) poly(alkoxy)dialkyl having the structure

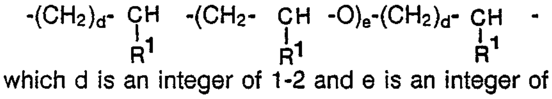

in which d is an integer of 1-2 and e is an integer of 0-350, (xvi) polyesters having the structure

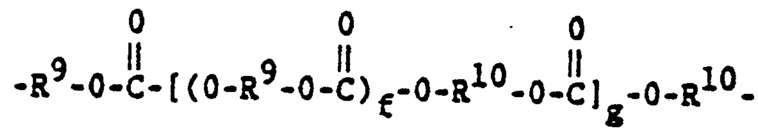

in which f and g are integers independently selected from 1-5, and $R^9$ and $R^{10}$ are independently selected from aliphatic or alicyclic diradical of 2-20 carbons, aromatic or araliphatic diradical of 6-22 carbons, alkyldiarylene of 13-20 carbons, and cycloalkyldialkyl of 7-12 carbons, (xvii) copolyesters having the structure

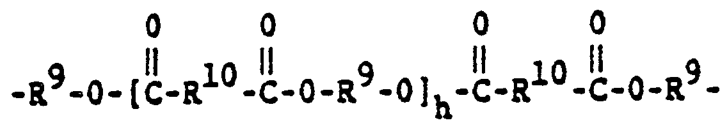

in which h is an integer from 0-10, (xviii) unsaturated polyolefins having the structure -[polybutadiene]- in which the polybutadiene has molecular weight of from 2000 to 3500, (xix) poly(mercaptoethers) having the structure

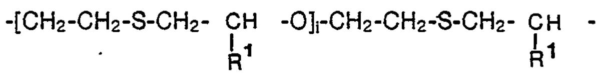

in which i is an integer from 2 to 12, (xx) unsaturated copolymers having the structure

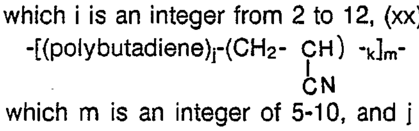

in which m is an integer of 5-10, and j and k are component fractions, k is selected from about 0.1 to 0.3 and j is 1-k, (xxi) bicyclic divalent hydrocarbons having the structure

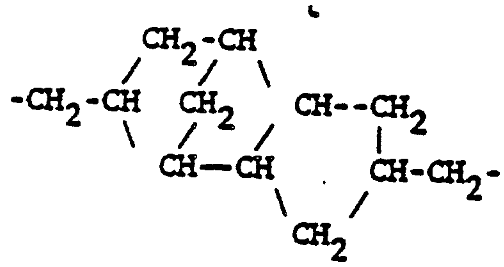

, and

(xxii) poly(organosiloxanes) having the structure

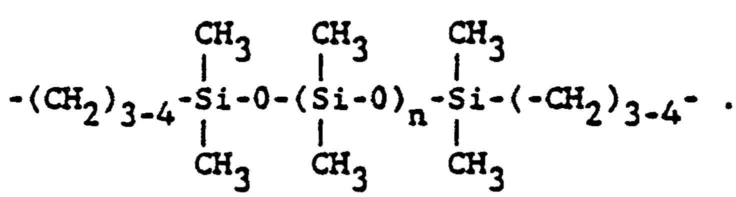

in which n is an integer of 5-3000.

6. The process of claim 1 for preparing a compound as in claim 3, wherein when s is at least one, $R^4$ is selected from

(i) polyester having the structure

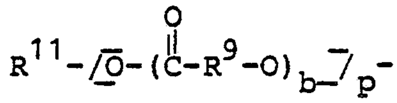

in which b is an integer of 1-10, p is 2 or 3, and when p is 2, $R^{11}$ is aliphatic diradical of 2 to 4 carbons, and when p is 3, $R^{11}$ is aliphatic triradical of 3 to 8 carbons, and

(ii) 4-aza-1,7-dioxaheptane-1,4,7-triyl,

(iii) 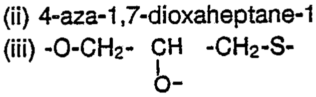

(iv)

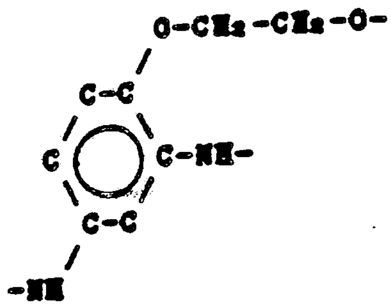

(v)

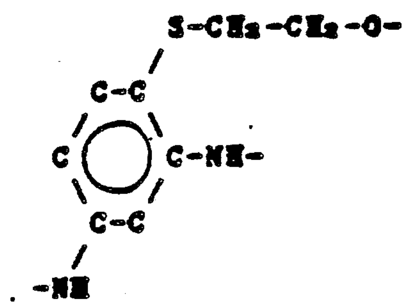

(vi)

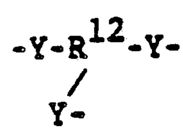

wherein

Y is as previously defined, $R^{12}$ is selected from aliphatic triradical of 3 to 20 carbons, aromatic triradical of 6 to 13 carbons, 1,3,5-triazine-2,4,6-triyl, triisopropylbenzene-alpha, alpha′, alpha″-triyl, nitrillotriethyl,

(vii)

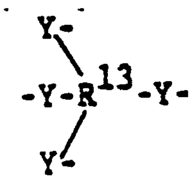

wherein

Y is previously defined, $R^{13}$ is aliphatic tetraradical of 4 to 20 carbons,

(viii)

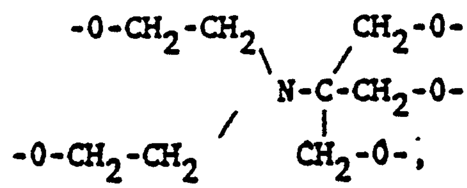

(ix)

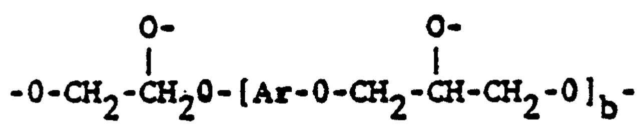 wherein

Ar is 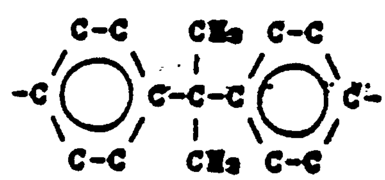

and b is as previously defined, and

(x) $-NH-(CH_2-CH_2-N)_{1-2}-CH_2-CH_2NH-$.

7. The process of claim 1 for preparing a compound as in claim 3, wherein when s is at least one, $R^4$ is selected from

(i)

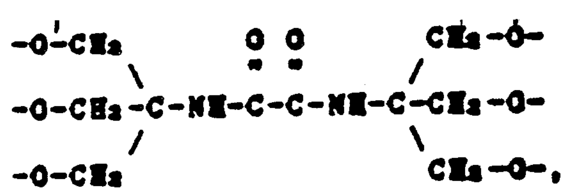

(ii)

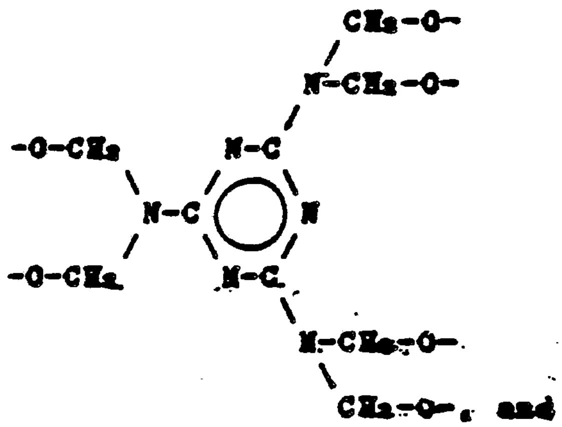

and

(iii)

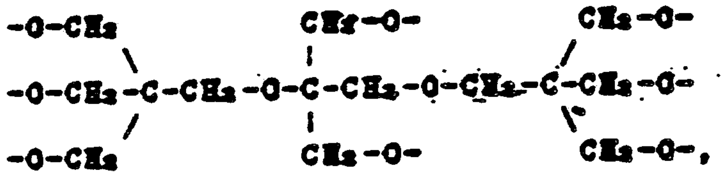

, and

(iv) a polymeric or copolymeric radical containing recurring units

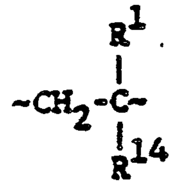

wherein

$R^{14}$ is selected from -O-, -N($R^6$)-, -$CH_2$-O-, -C(=O)-O-$CH_2$-CH(OH)-$CH_2$-O-, -C(=O)-O-$CH_2$-$CH_2$-O-, and -C(=O)-O-$CH_2$-$CH_2$-$CH_2$-O-, and ~ is the polymer or copolymer backbone in which the units recur.

8. The process of claim 1 for preparing a compound of the general formula (I) selected from N-(2,2,6,6-tetramethyl-4-piperidinyl)-4-carboxyphthalimide, N-(2,2,6,6-tetramethyl-4-piperidinyl)-4-(chlorocarbonyl)-phthalimide, hydrochloride salt, N-(2,2,6,6-tetramethyl-4-piperidinyl)-4-(aminocarbonyl)phthalimide, N-(2,2,6,6-tetramethyl-4-piperidinyl)-4-(ethoxycarbonyl)phthalimide, N-(2,2,6,6-tetramethyl-4-piperidinyl)-4-(2,2,6,6-tetramethyl-4-piperidinyloxycarbonyl)phthalimide, N-(2,2,6,6-tetramethyl-4-piperidinyl)-4-(2-ethylhexyloxycarbonyl)phthalimide, N-(2,2,6,6-tetramethyl-4-piperidinyl)-4-(dodecylaminocarbonyl)-phthalimide, N-(2,2,6,6-tetramethyl-4-piperidinyl)-4-(dibutylaminocarbonyl)phthalimide, N-(2,2,6,6-tetramethyl-4-piperidinyl)-4-(2,2,6,6-tetramethyl-4-piperidinylaminocarbonyl)phthalimide, styrene/N-(2,2,6,6-tetramethyl-4-piperidinyl)-4-(allyl-oxycarbonyl)phthalimide copolymer 4,4′-[2,5-dioxa-1,6-dioxohexane-1,6-diyl]bis[N-(2,2,6,6-tetramethyl-4-piperidinyl)phthalimide], 4,4′-[1,3-phenylenedi(oxycarbonyl)]bis[N-(2,2,6,6-tetramethyl-4-piperidinyl)-phthalimide], N-(2,2,6,6-tetramethyl-4-piperidinyl)-4-(octylphenoxypoly(ethoxy)carbonyl)-phthalimide, N-(2,2,6,6-tetramethyl-4-piperidinyl)-4-(allyloxycarbonyl)phthalimide, N-(2,2,6,6-tetra methyl-4-piperidinyl)-phthalimide-4-carbonyl endcapped amine terminated polypropylene glycol, N-(2,2,6,6-tetramethyl-4-piperidinyl)phthalimide-4-carbonyl endcapped polybutadiene diol, N-(2,2,6,6-tetramethyl-4-piperidinyl)-4-[(4-morpholinyl)carbonyl]phthalimide, N-(2,2,6,6-tetramethyl-4-piperidinyl)-4-{[4-(2,2,-di(methoxycarbonyl)-ethenyl)phenoxy]carbonyl}-phthalimide, N-(2,2,6,6-tetramethyl-4-piperidinyl)-4-{[2-hydroxy-3-(alkyl($C_{12}$-$C_{14}$)-oxy)propoxy]carbonyl}phthalimide, N-(2,2,6,6-tetramethyl-4-piperidinyl)-4-(butoxycarbonyl)phthalimide, N-(2,2,6,6-tetramethyl-4-piperidinyl)-4-(octadecyl-oxycarbonyl)phthalimide, N-(2,2,6,6-tetramethyl-4-piperidinyl)phthalimide-4-carbonyl endcapped amine terminated polypropylene oxide, N-(2,2,6,6-tetramethyl-4-piperidinyl)phthalimide-4-carbonyl endcapped aliphatic polycaprolactone diol, or N-(2,2,6,6-tetramethyl-4-piperidinyl)phthalimide-4-carbonyl endcapped poly(ethylene glycol) monomethyl ether, N-(2,2,6,6-tetramethyl-4-piperidinyl)phthalimide-4-carbonyl endcapped hydroxyterminated aliphatic polycarbonate, N-(2,2,6,6-tetramethyl-4-piperidinyl)-4-[(2-hydroxy-2-phenylethoxy)carbonyl]phthalimide, 4,4′-[4,13-di-hydroxy-1,16-dioxo-2,6,11,15-tetraoxa-hexadecane-1,16-diyl] bis[N-(2,2,6,6-tetramethyl-4-piperidinyl)-phthalimide, N-(1-acetyl-2,2,6,6-tetramethyl-4-piperidinyl)-4-(ethoxycarbonyl)phthalimide, 4,4′-(4-acetoxy-2,6-dioxa-1,7-dioxoheptane-1,7-diyl) bis[N-(1-acetyl-2,2,6,6-tetramethyl-4-piperidinyl)phthalimide], 4,4′-[2,5-dioxa-1,6-dioxohexane-1,6-diyl] bis[N-(2,2,6,6-tetramethyl-4-piperidinyl)phthalimide], and 4,4′-(4-acetoxy-2,6-dioxa-1,7-dioxoheptane-1,7-diyl) bis[N-(2,2,6,6-tetramethyl-4-piperidinyl)phthalimide].

9. Use of compounds prepared according to the process of one of claims 1 to 8 for stabilizing polymers and copolymers.

10. Composition on the basis of at least one polymer or copolymer, characterized by a content of at least one compound of claims 1 to 8.